# EUROPEAN PATENT SPECIFICATION

(11) **EP 1 877 038 B1**
(45) Date of publication and mention of the grant of the patent: **22.03.2017**
(21) Application number: 06758796.4
(22) Date of filing: 01.05.2006
(51) Int. Cl.: A61K 47/66, A61K 47/69, A61K 9/127, A61K 38/16, A61K 38/17, A61K 38/49, A61K 38/58, A61K 35/44, B82Y 5/00

(54) **CELL-SURFACE DECORATION WITH ACTIVE AGENTS**
ZELLOBERFLÄCHENDEKORATION MIT AKTIVEN AGENTIEN
DECORATION DE SURFACES DE CELLULES AVEC DES AGENTS ACTIFS

(30) Priority: 29.04.2005 US 676049 P
(43) Date of publication of application: 16.01.2008
(73) Proprietor: UNIVERSITY OF LOUISVILLE RESEARCH FOUNDATION, INC., Louisville, KY 40208 (US)
(72) Inventor: MALDONADO, Claudio, Louisville, Kentucky 40245 (US); GROSSI, Federico, V., Louisville, Kentucky 40241 (US); PEREZ-ABADIA, Gustavo, Louisville, Kentucky 40220 (US); EHRINGER, William, D., Charleston, Indiana 47111 (US)
(74) Representative: Isarpatent
(86) International application number: PCT/US2006/016480
(87) International publication number: WO 2006/119121

(56) References cited:
- WO-A2-02/02751
- WO-A2-2004/049907
- US-A- 4 429 008
- US-B1- 6 180 134
- CHIKH G G ET AL: "Attaching histidine-tagged peptides and proteins to lipid-based carriers through use of metal-ion-chelating lipids" BIOCHIMICA ET BIOPHYSICA ACTA. BIOMEMBRANES, AMSTERDAM, NL LNKD- DOI:10.1016/S0005-2736(02)00618-1, vol. 1567, 23 December 2002 (2002-12-23), pages 204-212, XP004399280 ISSN: 0005-2736
- SHAHINIAN S ET AL: "A novel strategy affords high-yield coupling of antibody Fab'fragments to liposomes" BIOCHIMICA ET BIOPHYSICA ACTA. BIOMEMBRANES, AMSTERDAM, NL LNKD- DOI:10.1016/0005-2736(95)00145-S, vol. 1239, 1 January 1995 (1995-01-01), pages 157-167, XP002974519 ISSN: 0005-2736
- MUZYKANTOV V R ET AL: "Avidin attachment to red blood cells via a phoshpolipid derivative of biotin provides complement-resistant immunoerythrocytes" JOURNAL OF IMMUNOLOGICAL METHODS, ELSEVIER SCIENCE PUBLISHERS B.V.,AMSTERDAM, NL LNKD- DOI:10.1016/0022-1759(93)90212-P, vol. 158, no. 2, 3 February 1993 (1993-02-03), pages 183-190, XP023974301 ISSN: 0022-1759 [retrieved on 1993-02-03]

## Description

### TECHNICAL FIELD

The presently disclosed subject matter relates generally to methods of decorating cells with active agents, and more particularly to methods of decorating cells using fusogenic lipid vesicles formulated with functionalized lipids that bind active agents with affinity.

### ABBREVIATIONS

- ACI: = August and Copenhagen-Irish (rat strain cross)
- APC: = antigen presenting cell
- CABG: = coronary artery by-pass grafting
- CPB: = cardiopulmonary by-pass
- CR1: = complement receptor 1
- CTLA4: = cytotoxic T-lymphocyte-associated protein 4
- DAF: = decay accelerating factor
- DGF: = delayed graph function
- DOPC: = 1,2-dioleoyl-sn-glycero-3-phosphocholine
- DOPC-e: = 1,2-dioleoyl-sn-glycero-3-ethylphosphocholine
- DOPE: = 1,2-dioleoyl-sn-glycero-3-phosphoethanolamine
- DOPS: = 1,2-dioleoyl-sn-glycero-3-[phospho-l-serine]
- DODAP: = 1,2-dioleoyl-3-dimethylammonium-propane
- DOTAP: = 1,2-dioleoyl-3-trimethylammonium-propane
- ECA: = external carotid artery
- EDTA: = ethylenediaminetetraacetic acid
- EJV: = external jugular vein
- FBS: = fetal bovine serum
- FUV: = fusogenic unilamellar vesicle
- GVHD: = graft-versus-host disease
- HBSS: = Hanks' balanced salts solution
- HUVEC: = human umbilical vein endothelial cell
- ICAM: = intercellular adhesion molecule
- IRI: = ischemia-reperfusion injury
- LCR: = ligase chain reaction
- LPS: = lipopolysaccharides
- MAC: = membrane attack complex
- MLR: = mixed lymphocyte reaction
- NPY: = neuropeptide Y
- PBS: = phosphate buffered saline
- PCR: = polymerase chain reaction
- PDPC: = 1-palmitoyl-2-docosahexaenoyl-sn-glycero-3-phosphocholine
- PEG: = polyethylene glycol
- POPA: = 1-palmitoyl-2-oleoyl-sn-glycero-3-phosphate
- SA: = streptavidin
- SPICE: = smallpox inhibitor of complement enzyme
- SUV: = small unilamellar vesicle
- SA-VCP: = streptavidin - VCP fusion protein
- SVG: = saphenous vein graft
- TNF: = tumor necrosis factor
- tPA: = tissue plasminogen activator
- UW: = University of Wisconsin Solution
- VCP: = vaccinia virus complement control protein
- WF: = Wistar-Furth (rat)

### BACKGROUND

Tissues that are subjected to prolonged ischemia and reperfusion injury (IRI) produce oxygen and nitrogen radicals, among other toxins, resulting in endothelial damage that can lead to inflammation, an increase in thrombogenic activity in arteries and veins, and an increase in the rate of acute rejection of allografts.

Ischemia can be caused by traumatic injury to vessels or by vascular occlusion intentionally induced in various clinical procedures, including transplantation of tissues and organs. IRI is considered a biphasic process. Ischemia initiates the injury by energy deprivation and metabolite accumulation and reperfusion worsens the damage by triggering an inflammatory reaction involving oxygen free radicals, (Halliwell, B. et al., 1992, FEBS Lett., 307:108-112; Kalayoglu, M. et al., 1988b, Lancet, 331:617-619; Lominadze, D. et al., 1997, J. Nutr., 127:1320-1327; Weisman, H. F. et al., 1990b, Science, 249:146-151; Wink, D. A. et al., 1994, Environ. Health Perspect., 102 Suppl 3:11-15) complement activation (Hill, J. H. et al., 1971d, 133:885-900) and leukocytes (Krishnaswamy, G. et al., 2001, Front Biosci., 6:D1109-D1127; Kvietys, P. R. et al., 2001, News Physiol Sci., 16:15-19). After blood flow is reestablished in ischemic tissues, oxygen is re-applied and repair mechanisms are set into action. During reperfusion, accumulated toxic metabolites are released into the systemic circulation and can adversely affect remote organs and/or the regenerative process in the ischemic organ.

Accumulating evidence suggests complement as a central mediator of reperfusion injury (Amsterdam, E. A. et al., 1995, 268:H448-H457; Hill, J. H. et al., 1971b, 133:885-900; Smith, G. W. et al., 1983, J. Clin. Lab Immunol., 12:197-199; Weiser, M. R. et al., 1996, 183:2343-2348). Complement activation in ischemic hearts was first reported by Hill et al. in 1971 (Hill, J. H. et al., 1971c, 133:885-900). Since then at least three separate complement pathways (the classical, lectin, and alternative pathways) have been identified to activate complement (Stahl, G. L. et al., 2003, 162:449-455). The classical pathway is activated by antigen-antibody interaction, which then leads to activation of complement component C1 q, followed by complement components C2- and C4-dependent cleavage of complement component C3 and, ultimately, cleavage of complement component C5 by formation of the classical C5 convertase (C4b2a3b). The alternative pathway is activated by the presence of lipopolysaccharide (LPS), and, to a certain extent, spontaneously generated C3b. In this pathway, C3b binds to factor B and forms a complex, which is cleaved by factor D to form the alternative C3 convertase, C3b(H₂0)Bb. Properdin acts as an amplifying activator and stabilizes this complex, enabling the cleavage product C3b to bind to it, thus forming the alternative C5 convertase (C3b3bBb). All pathways, therefore, use C3 and cleave C5, which results in the powerful pro-inflammatory cleavage products C5a and C5b-9 (Membrane Attack Complex, MAC). These two products of complement activation are believed to play a primary role in IRI (Mollnes, T. E. et al., 2002, Trends Immunol., 23:61-64), as confirmed in C5-deficient animals, which have reduced remote and local injury in models of IRI.

In autologous tissue transplantation, including for example saphenous vein grafts (SVGs), endothelial damage after IRI can induce thrombus formation which can lead to graft occlusion (Motwani, J. G. et al., 1998, Circulation, 97:916-931). Acute thrombosis of SVGs occurs in up to 12% of patients within 30 days after coronary artery by-pass grafting (CABG) (Bourassa, M. G., 1991, J. Am. Coll. Cardiol., 17:1081-1083; Fitzgibbon, G. M. et al., 1996, J. Am. Coll. Cardiol., 28:616-626). Endothelial disruption due to the physical handling of SVGs during harvest and/or due to factors associated with IRI, contribute heavily towards the activation of the coagulation cascade (Motwani, J. G. et al., 1998, 97:916-931). Again, ischemia initiates the injury by energy deprivation and accumulation of metabolic waste products, and reperfusion, exacerbates the damage by triggering inflammatory reactions involving oxygen free radicals, (Halliwell, B. et al., 1992, FEBS Lett., 307:108-112; Kalayoglu, M. et al., 1988a, Lancet, 331:617-619; Lominadze, D. et al., 1997, J.Nutr., 127:1320-1327; Weisman, H. F. et al., 1990a, Science, 249:146-151; Wink, D. A. et al., 1994, Environ.Health Perspect., 102 Suppl 3:11-15) complement activation (Hill, J. H. et al., 1971 a, 133:885-900) and leukocyte adherence (Krishnaswamy, G. etal., 2001, Front Biosci., 6:D1109-D1127; Kvietys, P. R. et al., 2001, News Physiol Sci., 16:15-1919). Furthermore, CABG in itself appears to disturb local factors influencing hemostasis, and during cardiopulmonary by-pass (CPB) plasma fibrinogen is elevated, favoring a prothrombotic response (Moor, E. et al., 1994, Thromb. Haemost., 72:335-342). CPB triggers a cascade of inflammatory mediators that further contribute towards endothelial disruption (Gu, Y. J. et al., 1998, Ann. Thorac. Surg., 65:420-424).

Intra-operative strategies to prevent SVG thrombosis include the "no touch technique" of handling tissues during harvest, (Tsui, J. C. et al., 2001, Br. J. Surg., 88:1209-1215) and the avoidance of SVG distention by insuring that infusion pressures never exceed 100 mmHg (Adcock, O. T., Jr. et al., 1984, Surgery, 96:886-894). SVG preservation after harvest using preservation solutions can also be used, although efficacy is controversial. However, organ preservation solutions have been tried experimentally with some success (Anastasiou, N. et al., 1997, J. Vasc. Surg., 25:713-721). University of Wisconsin solution (UW) has been reported to protect smooth cell function, (Cavallari, N. et al., 1997, Surgery, 121:64-71) and endothelial cell viability of SVGs (Barner, H. B. et al., 1990, J. Thorac. Cardiovasc. Surg., 100:148-149). It has been observed that UW primarily protects tissues against ischemia, but has little or no effect against reperfusion injury (Gao, W. et al., 1992, Transpl. Int., 5 Suppl 1:S329-S335). The use of hypothermia (4°C) to preserve SVGs has been shown to be detrimental to the endothelium, particularly, with physiological solutions. Grafts preserved at 20°C appear to have significantly less damage (Solberg, S. et al., 1987, J. Cardiovasc. Surg., 28:571-575). There is some consensus that SVGs should be stored at room temperature if they will be transplanted within 2 h, since the endothelium becomes fragile at low temperatures, (Solberg, S. et al., 1987, 28:571-575) and endothelium-dependent relaxation may become impaired.(Ingemansson, R. et al., 1996, Ann. Thorac. Surg., 61:1413-14171417)

In solid organ allografts IRI has been long implicated as a risk factor for the development of delayed graph function (DGF). DGF affects around 20% of renal transplants (Pirsch, J. D., 2002, Medscape Transplantation, 3:) increasing the amount of post-transplant interventions and extending hospitalization. Additionally, prolonged ischemia has been reported to contribute towards chronic allograft rejection in a long-term survival study in a rat model (Yilmaz, S. et al., 1992b, 53:823-827). In lung transplantation, preservation techniques have reduced early graft dysfunction; however, severe IRI still occurs in more than 10% of lung transplants (Yilmaz, S. et al., 1992a, 53:823-827). IRI has also been suggested as an alloantingen-independent factor influencing the occurrence of chronic allograft rejection (Tullius, S. G. et al., 1995, 59:313-318). Also, duration of ischemia and acute graft failure are two of several variables associated with early mortality (Bonet, L. A., 2003, 35:1946-1950) and chronic rejection (Baldwin, W. M., III et al., 1999, 68:894-900) in heart transplant patients. Since the introduction of University of Wisconsin Solution (UW) in 1987, no other clinically used solution has provided similar or better protection against ischemic injury and, although UW has become the gold standard for the preservation of intra-abdominal organs (Belzer, F. O. et al., 1992, Ann. Surg., 215:579-583) and has also been shown to protect the heart, (Swanson, D. K. et al., 1988, 7:456-467) it only protects tissues against ischemia, and has little or no effect against reperfusion injury (Gao, W. et al., 1992, Transpl. Int., 5 Suppl 1:S329-S335), as previously noted.

Transplanted allograft organs and tissues are also vulnerable to immune system rejection. In organ/tissue allografts, there are three distinct but overlapping phases in the process of transplant rejection: hyperacute, acute and chronic. Whereas the direct alloresponse seems to fade with time, the indirect alloresponse, fueled by a continuous supply of donor antigen to passenger leukocytes in the organ, appears to be indefinite.

Hyperacute rejection is almost immediate (e.g., during the first 48 hours) and is mediated by pre-formed antibodies in the recipient against MHC-I molecules on the graft's endothelium and/or blood-group antigens (A, B, Rh). This type of rejection is especially relevant in xenotransplantation. Acute and chronic rejections typically start around the third day post-transplantation and can last for years. Presumably, after an organ is transplanted, donor antigen presenting cells (APCs) migrate out of the organ into the recipient lymph nodes, which ultimately evokes a direct immune response against donor antigens in both helper and cytotoxic recipient T cells. This response, which dominates acute rejection, is thought to recruit many cross-reactive helper T cells, which are primed against various environmental antigens in the context of self-MHC (Lombardi et al., 1990, Int. Immunol. 2: 9-13; Merkenschlager et al., 1991, Eur. J Immunol., 21:79-88).

An indirect alloresponse is mediated by recipient APCs, which pick up donor antigens from dying donor APCs in the draining lymph nodes, but also directly from the organ (so-called passenger leukocytes), and present those primarily to recipient helper T cells. This indirect alloresponse is believed to be responsible for chronic rejection (Benichou et al., 1999, J Immunol., 162:352-358), although recent findings seem to indicate that this response also can contribute to acute rejection (Benham et al., 1995, Transplantation, 59:1028-1032; and Braun et al., 2001, J Immunol., 166:4879-4883).

WO2004/049907 discloses fusiogenic lipid vesicles comprising a biotinylated lipid for treating a transplant.

As such, there is a continuing unmet need for agents that can protect tissues and organs from IRI and immune system rejection during periods during and after accidental or induced ischemia, and during and after autologous, allograft, and xenograft transplantation.

### SUMMARY

In some embodiments of the presently disclosed subject-matter, a lipid vesicle is provided comprising a functionalized lipid comprising a tether moiety having binding affinity for a ligand portion of an active agent. The functionalized lipid comprises 1,2-dioleoyl-*sn*-glycero-3-{[N(5-amino-1-carboxypentyl)iminodiacetic acid]succinyl}. In some embodiments, the tether moiety is nickel. The ligand portion of the active agent is a poly-histidine. In some embodiments, the active agent is a polypeptide, an aptamer, or a small molecule. Further, in some embodiments the active agent is a therapeutic molecule selected from the group consisting of a T cell apoptosis-inducing molecule, a complement inhibitor, a T cell co-stimulatory blockade molecule, a leukocyte infiltration inhibitor, a neointimal hyperplasia inhibitor, an anticoagulant, and a thrombolytic. Still further, in some embodiments the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, factor IXa aptamer inhibitor 9.3tC, urokinase, tissue plasminogen activator (tPA), matrix metalloproteinases (MMP), neuropeptide Y (NPY) dummy receptors, and naturally occurring or synthetic glycoproteins or proteoglycans. In some embodiments, the lipid vesicle is lyophilized. In some embodiments, the lipid vesicle is a fusogenic lipid vesicle having a fusion rate of at least about 20 vesicle fusions/second/mm² of cell membrane.

Further, the lipid vesicle comprises a phospholipid which is a stable vesicle former; and at least one unstable vesicle forming member, wherein the unstable vesicle forming member is a polar lipid which is not a stable vesicle former. In some embodiments, the lipid vesicle has a ratio of the stable vesicle former to the functionalized lipid of from about 1:1 to about 500:1. As disclosed herein, the phospholipid which is a stable vesicle former or the polar lipid which is not a stable vesicle former has the structure of formula (I):

X-L-(Z)₂ (I),

wherein X is H, A, or has a structure of formula (II) 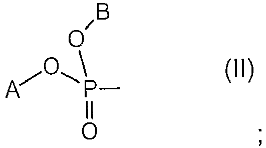
B is a cation or an alkyl group;
A is H or an alkyl group;
L is an alkyl group further missing two hydrogen atoms; and
each Z is independently H, E, or the structure of formula (XI), 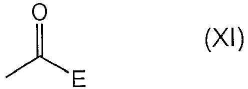 wherein E is an alkyl or alkenyl, and when one Z is H, the other Z is not H.
The lipid vesicle of claim 13, wherein A is H, or has a structure selected from the group consisting of formulas (III), (IV), (V), (VI) and (VII): 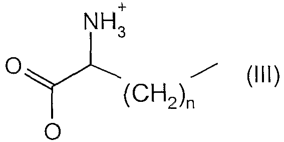 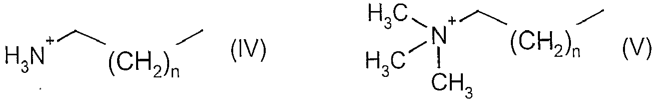 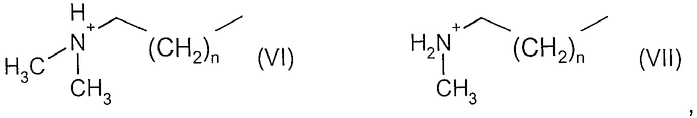
wherein n is an integer from 0 to 4;
L has a structure selected from the group consisting of formulas (VIII), (IX) or (X) 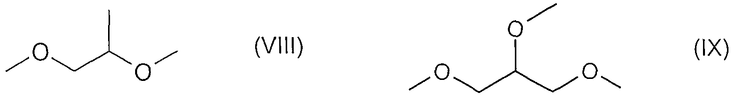 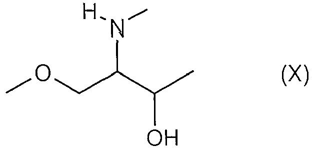 and
   E has a structure selected from the group consisting of (XII), (XIII), (XIV), (XV), (XVI), and (XVII)

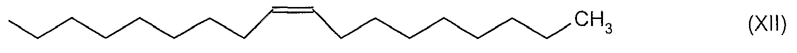

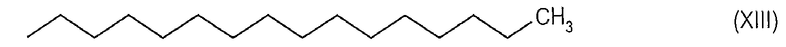

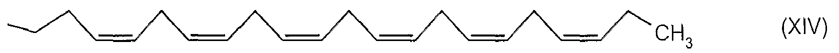

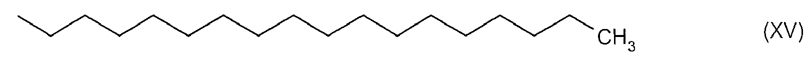

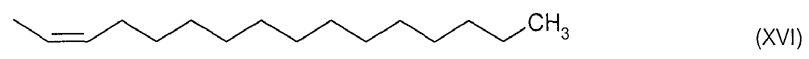

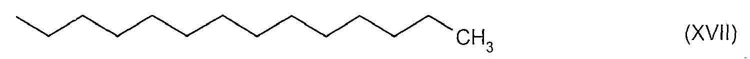

The phospholipid which is a stable vesicle former 1,2-dioleoyl-sn-glycero-3-phosphocholine (DOPC), 1-palmitoyl-2-docosahexaenoyl-sn-glycero-3-phosphocholine (PDPC), soy phosphatidylcholine, egg phosphatidylcholine, or a mixture thereof. The the unstable vesicle forming member is an unstable vesicle forming polar lipid selected from the group consisting of 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-l-serine] (DOPS),a sphingomyelin, 1,2-dimyristoyl-sn-glycerol, 1-palmitoyl-2-hydroxy-sn-glycero-3-phosphocholine, 1,2-dioleoyl-3-trimethylammonium-propane (DOTAP), and 1,2-dioleoyl-3-dimethylammonium-propane (DODAP).

In some embodiments of the presently disclosed subject matter, a kit for decorating endothelial cells is provided. In some embodiments, the kit comprises a lipid vesicle comprising a functionalized lipid comprising a tether moiety having binding affinity for a ligand portion of an active agent and the active agent comprising the ligand portion. In some embodiments, the kit comprises instructions for decorating endothelial cells. In some embodiments, the lipid vesicle is contained within a first container and the therapeutic molecule is contained within a second container.

In some embodiments of the presently disclosed subject matter, a method of decorating a cell membrane with an active agent is provided. In some embodiments, the method comprises contacting a cell with a lipid vesicle comprising a functionalized lipid comprising a tether moiety having binding affinity for a ligand portion of an active agent, wherein the tether moiety is nickel; and contacting the cell with the active agent comprising the ligand portion, wherein the ligand portion binds the tether moiety. In some embodiments, the cell is an endothelial cell. In some embodiments, the endothelial cell is an endothelial cell of a tissue or organ. In some embodiments, the tissue or organ is perfused with a first composition comprising the lipid vesicle and a second composition comprising the active agent. In some embodiments, the tether moiety is non-covalently bound to the ligand portion of the active agent. In some embodiments, the cell membrane is decorated with a plurality of different active agents.

In some embodiments of the presently disclosed subject matter, a lipid vesicle for use in a method of inhibiting rejection of a transplanted tissue or organ in a subject is provided. In some embodiments, the method comprises contacting the tissue or organ with a first composition comprising a lipid vesicle, wherein the lipid vesicle comprises a functionalized lipid comprising a tether moiety having binding affinity for a ligand portion of a therapeutic molecule, wherein the tether moiety is nickel; and contacting the tissue or organ with a second composition comprising the therapeutic molecule comprising the ligand portion, wherein the ligand portion binds the tether moiety. In some embodiments, the tissue or organ is contacted with the first and second compositions prior to transplant into the subject. In some embodiments, the tether moiety is non-covalently bound to the ligand portion of the therapeutic molecule.

In some embodiments of the presently disclosed subject matter, a lipid vesicle for use in a method of inhibiting ischemia-reperfusion injury to a tissue or organ is provided. In some embodiments, the method comprises contacting the tissue or organ with a first composition comprising a lipid vesicle, wherein the lipid vesicle comprises a functionalized lipid comprising a tether moiety having binding affinity for a ligand portion of a therapeutic molecule selected from the group consisting of complement inhibitors, anticoagulants, and thrombolytics; and contacting the tissue or organ with a second composition comprising the therapeutic molecule comprising the ligand portion, wherein the ligand portion binds the tether moiety. In some embodiments, the tether moiety is non-covalently bound to the ligand portion of the therapeutic molecule.

Accordingly, it is an object of the presently disclosed subject matter to provide methods of cell-surface decoration with therapeutic molecules and compositions related thereto. This object is achieved in whole or in part by the presently disclosed subject matter.

An object of the presently disclosed subject matter having been stated above, other objects and advantages will become apparent to those of ordinary skill in the art after a study of the following description of the presently disclosed subject matter and examples.

### DETAILED DESCRIPTION

The details of one or more embodiments of the presently disclosed subject matter are set forth in the accompanying description below. Other features, objects, and advantages of the presently disclosed subject matter will be apparent from the detailed description, and from the claims.

Cells, tissues and organs can be damaged due to IRI during and after periods of ischemia and reperfusion and/or immune system rejection after autologous, allograft, or xenograft transplantation. The presently disclosed subject matter provides materials and methods for inhibiting or treating damage to cells, tissues and organs due to IRI and/or transplantation. The presently disclosed subject matter provides compositions and methods for introducing exogenous therapeutic molecules into endogenous cell membranes by exploiting interactions, which can be strong and/or non-covalent, between tether moieties and ligands to which the tethers have binding affinity. The methods and materials of the presently disclosed subject matter avoid problems that can occur when trying to incorporate exogenous molecules directly into lipid membranes.

In some embodiments, the presently disclosed subject matter provides lipid vesicles comprising lipids modified with a tether moiety that has binding affinity for a specific ligand (*i.e.*, "functionalized lipids"). The presently disclosed subject matter further provides therapeutic molecules having a ligand portion to which the tether moiety has binding affinity. The lipid vesicle, when contacted with a cell, will fuse with the membrane of the cell, and thereby incorporate into the cell membrane (on the inner and/or outer surface of the cell membrane) the functionalized lipids, including the tether moiety. The treated cell can then be contacted with the therapeutic molecule, which will specifically bind to the cell membrane outer surface tether moiety by the ligand portion of the therapeutic molecule, resulting in decorating of the exterior surface of the cell with the therapeutic molecule. The cell can be an endothelial cell of a tissue or organ and the therapeutic molecule can be selected to provide to the cell (and surrounding tissue) protection against or treatment of damage to the cell and surrounding tissue due to ischemia and reperfusion and/or transplantation.

As such, the presently disclosed subject matter provides lipid vesicles comprising functionalized lipids, therapeutic molecules comprising ligands with binding specificity for the tether moieties of functionalized lipids, methods of decorating cells with the therapeutic molecules and cells thereby decorated, and methods of inhibiting rejection of a transplanted cell, tissue, or organ and/or methods of inhibiting IRI.

### I. Definitions

While the following terms are believed to be well understood by one of ordinary skill in the art, the following definitions are set forth to facilitate explanation of the presently disclosed subject matter.

Unless defined otherwise, all technical and scientific terms used herein have the same meaning as commonly understood to one of ordinary skill in the art to which the presently disclosed subject matter belongs. Although any methods, devices, and materials similar or equivalent to those described herein can be used in the practice or testing of the presently disclosed subject matter, representative methods, devices, and materials are now described.

Following long-standing patent law convention, the terms "a", "an", and "the" refer to "one or more" when used in this application, including the claims. Thus, for example, reference to "a cell" (*e.g*., "an endothelial cell") includes a plurality of such cells (*e.g*., a plurality of endothelial cells), and so forth.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed methods.

As used herein, "Alkyl" (or alkyl-or alk-) refers to a substituted or unsubstituted, straight, branched or cyclic hydrocarbon chain, preferably containing of from 1 to 20 carbon atoms. Suitable examples of unsubstituted alkyl groups include methyl, ethyl, propyl, isopropyl, cyclopropyl, butyl, iso-butyl, tert-butyl, sec-butyl, cyclobutyl, pentyl, cyclopentyl, hexyl, cyclohexyl, and the like. There can be optionally inserted along the alkyl chain one or more oxygen, sulfur or substituted or unsubstituted nitrogen atoms. "Alkylaryl" and "alkylheterocyclic" groups are alkyl groups covalently bonded to an aryl or heterocyclic group, respectively.

"Alkenyl" refers to a substituted or unsubstituted, straight, branched or cyclic, unsaturated hydrocarbon chain that contains at least one double bond, and preferably 2 to 22 carbon atoms. Exemplary unsubstituted alkenyl groups include ethenyl (or vinyl), 1-propenyl, 2-propenyl (or allyl) 1,3-butadienyl, hexenyl, pentenyl, 1,3,5-hexatrienyl, and the like. Preferred cycloalkenyl groups contain five to eight carbon atoms and at least one double bond. Examples of cycloalkenyl groups include cyclohexadienyl, cyclohexenyl, cyclopentenyl, cycloheptenyl, cyclooctenyl, cyclohexadienyl, cycloheptadienyl, cyclooctatrienyl and the like.

"Alkoxy" refers to a substituted or unsubstituted,-0-alkyl group. Exemplary alkoxy groups include methoxy, ethoxy, n-propoxy, isopropoxy, n-butoxy, t-butoxy, and the like.

"Aryl" refers to any monovalent aromatic carbocyclic or heteroaromatic group, preferably of 3 to 10 carbon atoms. The aryl group can be bicyclic (*i.e*., phenyl (or Ph)) or polycyclic (*i*.*e*. naphthyl) and can be unsubstituted or substituted. Preferred aryl groups include phenyl, naphthyl, furyl, thienyl, pyridyl, indolyl, quinolinyl or isoquinolinyl.

"Amino" refers to an unsubstituted or substituted-NRR' group. The amine can be primary (-NH₂), secondary (-NHR) or tertiary (-NRR'), depending on the number of substituents (R or R'). Examples of substituted amino groups include methylamino, dimethylamino, ethylamino, diethylamino, 2-propylamino, 1-propylamino, di(n-propyl)amino, di(iso-propyl)amino, methyl-n-propylamino, t-butylamino, anilino, and the like.

"Heterocyclic radical" refers to a stable, saturated, partially unsaturated, or aromatic ring, preferably containing 5 to 10, more preferably 5 or 6, atoms. The ring can be substituted 1 or more times (preferably 1, 2, 3, 4 or 5 times) with a substituent. The ring can be mono-, bi-or polycyclic. The heterocyclic group consists of carbon atoms and from 1 to 3 heteroatoms independently selected from the group consisting of nitrogen, oxygen, and sulfur. The heteroatoms can be protected or unprotected. Examples of useful heterocyclic groups include substituted or unsubstituted, protected or unprotected acridine, benzathiazoline, benzimidazole, benzofuran, benzothiophene, benzthiazole, benzothiophenyl, carbazole, cinnoline, furan, imidazole, 1 H-indazole, indole, isoindole, isoquinoline, isothiazole, morpholine, oxazole (*i*.*e*., 1,2,3-oxadiazole), phenazine, phenothiazine, phenoxazine, phthalazine, piperazine, pteridine, purine, pyrazine, pyrazole, pyridazine, pyridine, pyrimidine, pyrrole, quinazoline, quinoline, quinoxaline, thiazole, 1,3,4-thiadiazole, thiophene, 1,3,5-triazines, triazole (*i.e.*, 1,2,3-triazole), and the like.

"Substituted" means that the moiety contains at least one, preferably 1-3 substituent(s). Suitable substituents include hydrogen (H) and hydroxyl (--OH), amino (-NH₂), oxy (-O-), carbonyl (-CO-), thiol, alkyl, alkenyl, alkynyl, alkoxy, halo, nitrile, nitro, aryl and heterocyclic groups. These substituents can optionally be further substituted with 1-3 substituents. Examples of substituted substituents include carboxamide, alkylmercapto, alkylsulphonyl, alkylamino, quaternary nitrogen, dialkylamino, carboxylate, alkoxycarbonyl, alkylaryl, aralkyl, alkylheterocyclic, and the like.

The term "binding affinity" refers to an affinity between two molecules, for example, a tether moiety and a ligand. Thus, as used herein, "binding affinity" refers to a preferential binding of one molecule with another in a mixture of molecules. In some embodiments, the binding of a ligand to a tether moiety can be considered specific if the binding affinity is about 1 x 10⁴ M⁻¹ to about 1 x 10⁶ M⁻¹ or greater. The phrases "specifically (or selectively) binds" and "binds with specificity", when referring to the binding capacity of a ligand or a tether moiety, refers to a binding reaction which is determinative of the presence of the respective target in a heterogeneous population of proteins and other biological materials.

The term "inhibitor" refers to a chemical or biological substance that inhibits, that is inactivates or decreases, the biological activity of a molecule, polypeptide (such as for example a complement component), or cell (such as for example a T cell).

A "functionalized lipid" is a lipid that, in addition to its native structure, contains a molecular modification or addition. The addition can be added covalently or non-covalently (*e.g*., by chelation). Such modifications include the addition of small molecules (*e.g*., biotin or FITC), polysaccharides, heparin, and elements (*e.g*., nickel).

The term "nucleic acid" refers to deoxyribonucleotides or ribonucleotides and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof *(e.g*., degenerate codon substitutions) and complementary sequences and as well as the sequence explicitly indicated. Specifically, degenerate codon substitutions can be achieved by generating sequences in which the third position of one or more selected (or all) codons is substituted with mixed-base and/or deoxyinosine residues (Batzer et al. (1991) Nucleic Acid Res 19:5081; Ohtsuka et al. (1985) J Biol Chem 260:2605-2608; Rossolini et al. (1994) Mol Cell Probes 8:91-98). The terms "nucleic acid" or "nucleic acid sequence" can also be used interchangeably with gene, open reading frame (ORF), cDNA, and mRNA encoded by a gene.

The terms "polypeptide", "protein", and "peptide", which are used interchangeably herein, refer to a polymer of the 20 protein amino acids, or amino acid analogs, regardless of its size or function. Although "protein" is often used in reference to relatively large polypeptides, and "peptide" is often used in reference to small polypeptides, usage of these terms in the art overlaps and varies. The term "polypeptide" as used herein refers to peptides, polypeptides, and proteins, unless otherwise noted. The terms "protein", "polypeptide" and "peptide" are used interchangeably herein when referring to a gene product. Thus, exemplary polypeptides include gene products, naturally occurring proteins, homologs, orthologs, paralogs, fragments and other equivalents, variants, and analogs of the foregoing.

The terms "transformed", "transgenic", and "recombinant" refer to a cell of a host organism such as a mammal into which a heterologous nucleic acid molecule has been introduced. The nucleic acid molecule can be stably integrated into the genome of the cell or the nucleic acid molecule can also be present as an extrachromosomal molecule. Such an extrachromosomal molecule can be auto-replicating. Transformed cells, tissues, or subjects are understood to encompass not only the end product of a transformation process, but also transgenic progeny thereof. A "non-transformed," "non-transgenic", or "non-recombinant" host refers to a wild type organism, *e.g*., a mammal or a cell therefrom, which does not contain the heterologous nucleic acid molecule.

A "transplant" is any cell, tissue, appendage or organ that is transferred from a donor subject to a recipient subject. In the case of autologous transplants, the donor and recipient are the same subject. Examples of transplants include sperm, eggs, platelets, blood, skin, muscle, adipose tissue, nerve tissue, blood vessels, lymph, bone, ligament, eye, tongue, lung, trachea, heart, spleen, stomach, intestine, kidney, liver, finger, hand, toe, foot, arm and leg.

### II. Lipid Vesicles

In some embodiments of the presently disclosed subject matter, lipid vesicles are provided that can be utilized for delivery of tether moieties that bind active agents (*e.g*., therapeutic molecules) of interest to the cell membranes of target cells, and in some embodiments, to the outer cell membrane surface of target cells. Tether moieties, once incorporated into target cell membranes, provide for targeted delivery and "decoration" of cells with active agents to which the tether moieties have binding affinity. Further, the density of decoration on a cell can be controlled by changing the concentration of tether moieties in the vesicle's formulation, by changing the number of vesicles in a solution, and/or by changing the density of vesicles in a solution.

Lipid vesicles (*e.g*., liposomes) can be used to deliver molecules, including for example drugs such as antibiotics and anticancer agents. Vesicles can be filled with a variety of agents, which can then be delivered to target cells. This delivery method can be advantageous, as it sequesters the molecule within the vesicle until delivery to a cell, thereby protecting non-targeted cells from exposure to the encapsulated molecules if toxic (such as certain anticancer agents). Further, lipid vesicles are generally non-toxic because of their similarity to cell membranes. They can also protect their cargo from being diluted or degraded in the blood.

Vesicles can be comprised of phospholipids (amphipathic molecules) that form closed, fluid-filled spheres when mixed in aqueous solutions. As a lipid vesicle forms, the water-soluble molecules in the solution are encapsulated into the aqueous space in the interior of the sphere, whereas the lipid-soluble molecules in the solution are incorporated into the lipid bilayer. Thus, besides incorporating molecules into the interior of a lipid vesicle, molecules of interest can also be incorporated into the lipid membrane of the vesicle, resulting in delivery of lipid-soluble and amphipathic molecules to the cell membrane of a target cell.

Interactions between lipid vesicles and cell membranes can take several forms. Lipid vesicles can adsorb to almost any cell type. Once they have adsorbed, the spheres may be endocytosed by some cells. Adsorbed lipid vesicles can also exchange lipids with cell membranes and can in certain instances fuse with cells. The particular composition of the vesicle can affect whether or not the vesicle is able to fuse with a cell membranes. "Lipid vesicle" as the term is used herein, includes lipid vesicles that fuse with cell membranes (*i.e.*, "fusogenic vesicles"). When fusion takes place, the vesicle membrane is integrated into the cell membrane and the aqueous contents of the lipid vesicle merge with the fluid in the cytosol. Because individual lipid vesicles are capable of carrying thousands of therapeutic molecules, this technique can be desirable for delivering otherwise impermeable substances to cells.

A lipid vesicle can be made in a variety of layers and sizes. Multilamellar vesicles can be constructed and have multiple layers of lipid and water, whereas unilamellar vesicles have a single phospholipid bilayer. To engineer small fusogenic vesicles that effectively deliver their lipid membrane component, the vesicles must not fuse with each other, but must still be able to fuse with the membrane of target cells. To enhance vesicle fusion to cell membranes, the charge of the phospholipid head group can be manipulated to create dissimilar regions in the lipid layer. In addition, the vesicle's radius of curvature can be adjusted to provide different levels of stored kinetic energy. Multilamellar vesicles are generally not readily fusogenic, in part because the stored energy of the vesicle's radius of curvature is minimal. However, small unilamellar vesicles (SUVs), which have a very tight radius of curvature, are very fusogenic. Without wishing to be limited by theory, it has been reported that changes in lipid composition can dramatically alter vesicle-cell membrane fusion rates, and it has been determined that by tightening the vesicle's radius of curvature, the fusion rate can be further increased.

Methods for making fusogenic unilamellar vesicles (FUVs) are known in the art. FUVs can be made using various methods including sonication, homogenization or by the freeze and thaw method. See, for example, U.S. Patent Publication Nos. 2004/0213766 and 2003/0235611; U.S. Patent No. 6,417,326, Szoka et al., Ann. Rev. Biophys. Bioeng. 9:467 (1980); U.S. Pat. Nos. 4,186,183, 4,217,344, 4,235,871, 4,261,975, 4,485,054, 4,501,728, 4,774,085, 4,837,028, 4,946,787; PCT Publication No. WO 91/17424; Szoka & Papahadjopoulos, Proc. Natl. Acad. Sci. USA 75: 4194 4198 (1978); Deamer & Bangham, Biochim. Biophys. Acta 443: 629 634 (1976); Fraley et al., Proc. Natl. Acad. Sci. USA 76: 3348 3352 (1979); Hope et al., Biochim. Biophys. Acta 812: 55 65 (1985); Mayer et al., Biochim. Biophys. Acta 858: 161 168 (1986); Williams et al., Proc. Natl. Acad. Sci. USA 85: 242 246 (1988), Liposomes, ch. 1 (Ostro, ed., 1983); and Hope et al., Chem. Phys. Lip. 40: 89 (1986).

The presently disclosed subject matter provides lipid vesicles comprising modified lipids having a tether moiety attached thereto. The modified lipid comprising the tether moiety, also referred to herein as a "functionalized lipid", can be incorporated as a lipid component of the lipid vesicle. The tether moiety has binding affinity for a specific ligand. The ligand can be a natural part of, or incorporated into (*e.g*., a fusion or chimeric polypeptide) an active agent (for example a therapeutic molecule). As such, the functionalized lipid can function to bind with affinity the active agent through the tether moiety on the functionalized lipid and the ligand portion of the active agent. Accordingly, an active agent can be targeted to a cell membrane surface by first contacting the lipid vesicle comprising the modified lipid with the cell to allow the vesicle to fuse with the cell membrane and incorporate the functionalized lipid (and tether moiety) into the cell membrane, and then contacting the cell with the active agent. The therapeutic molecule binds with affinity to the tether moiety incorporated into the cell membrane, decorating the targeted cell surface with the therapeutic molecule. The active agent can then function as intended on the decorated cell(s) and/or on cells, tissues and organs within the vicinity of the decorated cell(s).

By using lipid vesicles to incorporate functionalized lipids within the membrane of target cells, an active agent including a ligand specifically binds the tether moiety and remains tethered to the surface of a cell for significantly longer periods of time than when non-specifically bound tethers (not incorporated into functionalized lipids) is used to attach molecules to the surface of a cell. In addition, because the functionalized lipids are not attached to the cytoskeleton, they have much more lateral freedom in the external leaflet of cell membranes. This permits the "floating" functionalized lipids to easily approach each other and the active agents are free to move as needed to function properly. For example, the trimerization necessary for FasL functionalization is facilitated considerably utilizing the lipid vesicles of the presently disclosed subject matter to incorporate tether moieties into target cell membranes.

The tether moiety is selected based on its compatibility with the functionalized lipid along with consideration for the ligand to which the tether moiety has binding affinity. For example, the tether can be nickel (using the nickel-chelating group N",N"-bis[carboxymethyl]-L-lysine (nitriloacetic acid) (NTA)).

The ligand portion of the active agent can be a natural part of the active agent, that is, it is found as a component of the functioning active agent without additional manipulation, or the ligand can be incorporated into the active agent. For example, if the active agent is a therapeutic polypeptide, certain ligands can be incorporated into the therapeutic polypeptide through recombinant genetic techniques, as is generally understood by one of skill in the art. The ligand portion of the active agent is a poly-histidine.

Also disclosed herein are functionalized lipids comprising a phospholipid, such as for example a phosphoethanolamine. Exemplary phospholipids disclosed herein include N-4-(p-maleimidophenyl)-butyryl dipalmitoylphosphatidylethanolamine (MPB-DPPE), N-4-(p-maleimidophenyl)-butyryldimyristoylphosphatidylethanolamine (MPB-DMPE), and N-4-(p- maleimidophenyl)-butyryl egg phosphatidylethanolamine (MPB-EPE). As disclosed herein, the tether moiety is biotin and the functionalized lipid is a phosphoethanolamine. The biotin can be covalently linked to the functionalized lipid. The biotin tether moiety binds with affinity to streptavidin and avidin ligands. As disclosed herein, the functionalized lipid including the tether moiety is 1,2-dioleoyl-*sn*-glycero-3-phosphoethanolamine-N-(biotinyl), 1,2-dipalmitoyl-*sn*-glycero-3-phosphoethanolamine-N-(biotinyl), and 1,2-dihexadecanoyl-*sn*-glycero-3-phosphoethanolamine-N-(biotinyl). Each of these exemplary functionalized lipids, and other comparable lipids are commercially available from for example Avanti Polar Lipids, Inc. (Alabaster, Alabama, U.S.A.) and/or Molecular Probes (Eugene Oregon, U.S.A.).

The functionalized lipid of the present invention is 1,2-dioleoyl-*sn*-glycero-3-{[N(5-amino-1-carboxypentyl)iminodiacetic acid]succinyl}. In particular embodiments, the functionalized lipid including the tether moiety is 1,2-dioleoyl-*sn*-glycero-3-{[N(5-amino-1-carboxypentyl)iminodiacetic acid]succinyl} (nickel salt) (Avanti Polar Lipids, Inc.). The nickel tether moiety is chelated to the hydrophilic head group of the functionalized lipid. The nickel tether moiety has binding affinity to the poly-histidine ligand, which can be incorporated into therapeutic molecules, for example therapeutic polypeptides.

Cells can be decorated with any one of a number of active agents, including for example therapeutic molecules, using the lipid vesicles disclosed herein to anchor tether moieties onto the surface of target cell membranes. "Therapeutic molecule", as the term is used herein, refers to molecules that can reduce or prevent cellular injury due to IRI and/or organ transplantation, and includes, for example polypeptides, aptamers, and small molecules. For example, molecules that can dampen a response from an arm of the immune system, including complement-mediated immune responses and leukocyte-mediated immune responses, as well as anticoagulants and thrombolytics are all exemplary of therapeutic molecules of the presently disclosed subject matter. Thus, the therapeutic molecules of the presently disclosed subject matter can be used to treat, for example, IRI, hyper-acute rejection, delayed graft function, chronic rejection, or late graft failure. Classes of therapeutic molecules that can be used to inhibit, prevent, or treat cellular damage associated with IRI and/or organ and tissue transplantation include, but are not limited to, T cell apoptosis-inducing molecules, complement inhibitors, T cell co-stimulatory blockade molecules, leukocyte infiltration inhibitors, naturally occurring or synthetic glycoproteins or proteoglycans, anticoagulants, thrombolytics, and neointimal hyperplasia inhibitors.

For example, with regard to neointimal hyperplasia inhibitors, neuropeptide Y (NPY) regulates cardiovascular function, smooth muscle contraction and smooth muscle cell proliferation. Blockade of the NPY Y1 receptor with for example BIBP3226 ((R)-N2-(diphenylacetyl)-N-[(4-hydroxy-phenyl)methyl]-D-arginine-amide) reduces mitogen-activated protein kinase (MAPK) activity in smooth muscle cells, and reduces neointimal hyperplasia after angioplasty. Display of "dummy" NPY Y1 receptors without the intracellular signaling component will bind NPY protein and competitively inhibit the activation of endogenous NPY Y1 receptors, thus, reducing neointimal hyperplasia. Similarly, since activation of NPY Y2 receptors have also been associated with neointimal hyperplasia but to a lesser extent than NPY Y1 s, the display of NPY Y2 dummy receptors also could be used for the same purpose.

For example, T cell apoptosis-inducing molecules can be used to decorate cells. An "apoptosis-inducing molecule" is a molecule that when contacted with a cell, induces apoptosis in that cell. The molecule can be a polypeptide, an organic molecule, a lipid, a hormone, etc., or a combination of such molecules (or moieties or fragments thereof). The molecule can be modified from its natural state; for example, a polypeptide that is an apoptosis-inducing molecule can be modified by either post-translational modifications, or using recombinant technology, to create, for example, chimeric (fusion) molecules.

Fas and FasL are two proteins that interact to activate one of the best defined apoptotic (programmed cell death) pathways. Fas and FasL are both members of the TNF (tumor necrosis factor) family. Fas is part of the transmembrane receptor family and FasL is part of the membrane-associated cytokine family. Three different forms of FasL are known: membrane-bound, soluble, and vesicular. Each differs in their function with respect to apoptosis and immune regulation. Apoptosis is primarily mediated by the vesicular and membrane-bound forms of FasL, whereas the soluble form is ineffective in mediating apoptosis and serves as an anti-apoptotic factor by competing for the Fas receptor with membrane bound FasL (Schneider et al., 1998, J. Exp. Med., 187:1205-1213.; Suda et al., 1993, Cell, 75:1169-1178). The apoptotic activity of FasL is most efficiently mediated by its trimerization and through cell-to-cell contact, which is counterbalanced by the anti-apoptotic activity of the soluble form. The four known main roles of Fas binding to pro-apoptotic FasL are: (1) for CD4 T cells to maintain lymphocyte homeostasis; (2) for triggering the death of macrophages infected with bacteria; (3) for killing anergic B cells; and (4) for CD8 T cells to kill virally infected target cells (Janeway et al., 2001, Immunobiology, Garland Publishing, New York, N. Y.). Several studies have demonstrated effective blockade of alloreactive responses and survival of allogeneic liver, kidney, thyroid, and pancreatic islets using FasL expressed via genetic modifications as an immunomodulatory approach (see, e.g., U.S. Published Patent Application No. 2004/0213766).

Therapeutic molecules that block complement activation can be used to decorate cells as well. Representative therapeutic molecules that block complement activation include but are not limited: to decay accelerating factor (DAF), vaccinia virus complement control protein (VCP), a polypeptide produced by vaccinia virus (See U.S. Patent Nos. 5,157,110 and 5,187,268 ); complement receptor 1 (CR1), a human anti-complement protein; smallpox inhibitor of complement enzymes (SPICE), a homologue of VCP produced by the small pox virus; and compstatin, an inhibitor of C3. Representative therapeutic small molecules complement inhibitors include but are not limited to complement hemolysis inhibitors FUT-175 and compound 4077, and the C1s inhibitors C1s-INH-248 and compound 53 (3-Dimensional Pharmaceuticals, Yardley, Pennsylvania, U.S.A.). VCP, for example, has been shown to inhibit both the classical and the alternative pathways of complement activation. Such complement inhibiting molecules serve as therapeutic molecules against inflammatory (including IRI) and autoimmune diseases. VCP is a 28 kDa protein with structural and functional similarities to the human proteins of the complement control family (C4b-BP and CR1). VCP binds C3b and C4b in the presence of co-factor I and blocks formation of C3 convertase complex, a crucial step in the synthesis of the potent chemotactic factor C5a and formation of the MAC. In addition to VCP, other members of the Poxvirus family encode homologues to VCP, including but not limited to SPICE, which can also be utilized with the presently disclosed subject matter as therapeutic molecules.

In addition, therapeutic molecules involved in co-stimulatory blockade (*i*.*e*., T cell co-stimulatory blockade molecules) can be used to decorate cells. Representative therapeutic molecules involved in co-stimulatory blockade include but are not limited to cytotoxic T-lymphocyte-associated protein 4 (CTLA4), which blocks the CD28-B7 pathway, and anti-CD40L, which blocks the CD40--CD40L pathway.

Representative therapeutic molecules that prevent leukocyte infiltration (*i.e*., leukocyte infiltration inhibitors) include but are not limited to naturally occurring or synthetic glycoproteins and proteoglycans. Glycoproteins or proteoglycans can cover or tower over other cell adhesion molecules (e.g., selectins, ICAMs, or integrins), which are essential for leukocyte rolling, adhesion, and translocation across the endothelium. Exemplary glycoproteins and proteoglycans useful as leukocyte infiltration inhibitors include but are not limited to inactive oversized lectins (*e.g*., L, P, and E selectins), and *α*-1-acid glycoprotein (AGP). AGP inhibits lymphocyte proliferation and neutrophil activation by interacting with the surface of their cell membranes.

Representative anticoagulants includes but are not limited to hirudin, both recombinantly produced or isolated from natural sources, small molecule factor Xa inhibitors: DPC-423, DPC-602, razaxaban, GSK 813893, otamixaban, DU-176b, KFA-1982, BAY-59-7939, DX-9065a, YM-150, LY-517717, MCM09; small molecule thrombin inhibitors: SSR-182289, LB-30057, LB-30870, BIBR-1048, L-374,087; and factor IXa aptamer inhibitor 9.3tC.

Representative thrombolytics include but are not limited to urokinase, tissue plasminogen activator (tPA) (including recombinant tPA) and matrix metalloproteinases (MMPs) (*e.g*., including: gelatinases (MMP-2,9), collagenases (MMP-1,8,13,14,18), and membrane bound MMPs (MMP-14,15,16,17,23,24,25)).

The lipid vesicle comprises a phospholipid which is a stable vesicle former; at least one unstable vesicle forming member, wherein the unstable vesicle forming member is a polar lipid which is not a stable vesicle former; and a functionalized lipid as disclosed herein. An unstable vesicle forming member is a molecule that decreases the stability of a vesicle, which can then advantageously increase the capacity of the vesicle to fuse with cell membranes (*i.e*., increase the fusogenicity of the vesicle).

Polar lipids are organic molecules which have a hydrophobic end and a hydrophilic end, and contain at least six carbon atoms; they have the structure of formula (I), where X is a head group, L is a back bone group, and each Z is a fatty group. The two Z groups may be the same or different. A phospholipid is a polar lipid which has a head group of formula (II), where A and B are substituents of the head group.

X-L-(Z)₂ (I)

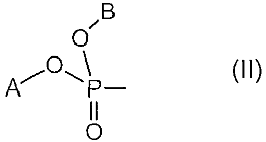

The head group, X, can be any polar group, preferably a cationic, anionic or zwitterionic group, or H. More preferably X is a group of formula (II). B can be a cation, such as Na⁺, K⁺, or tetramethyl ammonium ion; or an alkyl group. A can be H, or an alkyl group, and in some embodiments, A is an alkyl group substituted with an amine, such as for example a group of formula (III), (IV), (V), (VI) or (VII). It should be noted that throughout the specification, the formulas can show the structures in protonated form, but that they also include the unprotonated form (and vice versa); which form is present in any composition will depend on the exact pH of the composition, and the presence of water and/or appropriate counter ions.

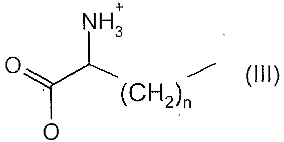

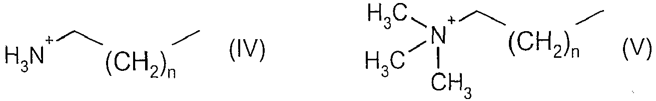

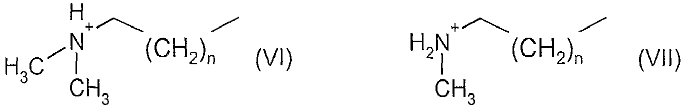

The back bone group, L, can be an alkyl further missing two hydrogen atoms (to give a total of three open attachment points), in some embodiments an alkoxy, or amino substituted alkyl. In particular embodiments, L is a group of formula (VIII), (IX) or (X).

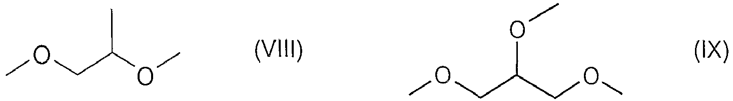

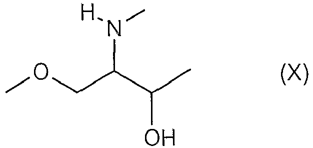

The fatty groups, Z, can be the same or different, and are H, an E group, or the structure of formula (XI), where E is an alkyl or alkenyl. In certain embodiments, E is an unsubstituted straight chain alkyl or alkenyl, with 6-26 carbon atoms. For example, E can be a group of formula (XII), (XIII), (XIV), (XV), (XVI), or (XVII). If one of the fatty groups is H, then the other must be different. If double bands are present, then cis configuration is preferable.

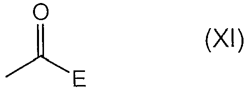

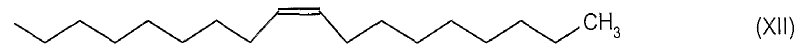

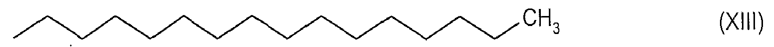

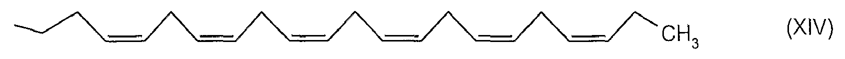

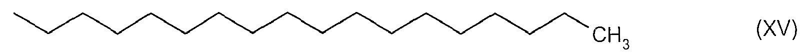

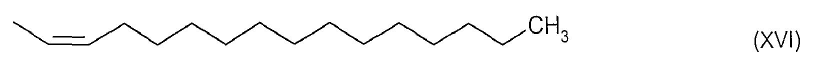

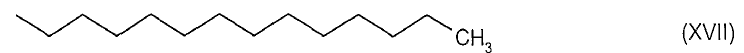

A phospholipid (or polar lipid) which is a stable vesicle former is a phospholipid (or polar lipid) that will form vesicles, at least 50% of which persist for at least one hour, when prepared as follows: the phospholipid is dissolved in chloroform and placed in a glass test tube. Solvent is removed by evaporation under a steady stream of nitrogen, followed by air removal by subjecting the sample to vacuum for twelve hours. The dried lipid material is then re-hydrated in 10 mM Na₂HPO₄, for 60 minutes at a temperature above the lipid phase transition temperature; the desired final concentration is 25 mg/ml. The lipid mixture is then agitated by sonication with a microtip 450 watt sonicator used at a 40% duty cycle.

In addition to the phospholipid which is a stable vesicle former, at least one other polar lipid is included (along with the functionalized lipid) in the lipid vesicle, which is an unstable vesicle forming member.

A raft former is a compound which will sit within the lipid layer of a vesicle when the vesicle is in an aqueous solution, and will form or cause formation of discrete regions within the vesicle wall (also known as rafts). These discrete regions tend to destabilize the vesicle, increasing its fusogenicity. Examples of raft formers are cholesterol, sphingomyelin, and proteins and polypeptides know to be membrane bound. Fusogenicity can also be enhanced by selecting polar lipids, which will result in a surface charge on the vesicle, which is the opposite of the charge of the Gouey-Chapman layer of the target cells (typically the Gouey-Chapman layer is positively charged).

As disclosed herein, the phospholipid which is a stable vesicle former or the polar lipid which is not a stable vesicle former has the structure of formula (I):

X-L-(Z)₂ (I),

wherein X is H, A, or has a structure of formula (II) 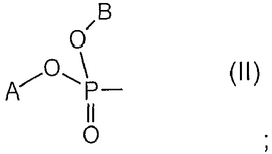
B is a cation or an alkyl group;
A is H or an alkyl group;
L is an alkyl group further missing two hydrogen atoms (to give a total of three open attachment points), and in some embodiments L is an alkoxy, or amino substituted alkyl; and
each Z is independently H, E, or the structure of formula (XI), 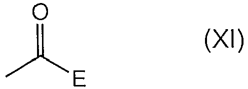 wherein E is an alkyl or alkenyl, and when one Z is H, the other Z is not H.

In some embodiments, A is H, or has a structure selected from the group consisting of formulas (III), (IV), (V), (VI) and (VII): 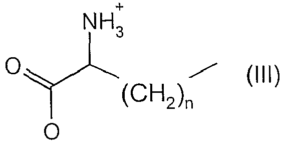 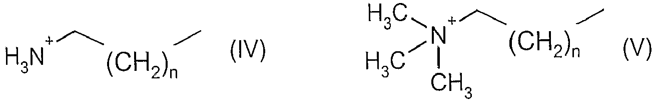 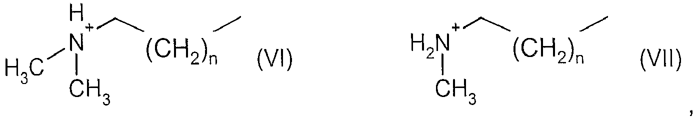
wherein n is an integer from 0 to 4;
L has a structure selected from the group consisting of formulas (VIII), (IX) or (X) 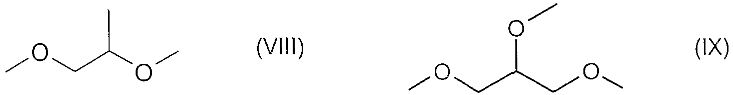 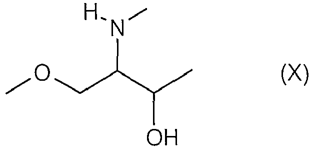 and
   E has a structure selected from the group consisting of (XII), (XIII), (XIV), (XV), (XVI), and (XVII)

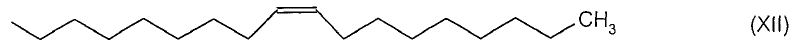

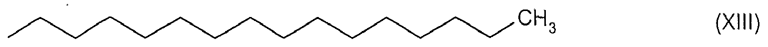

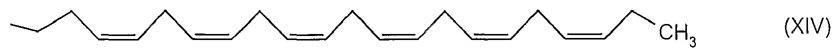

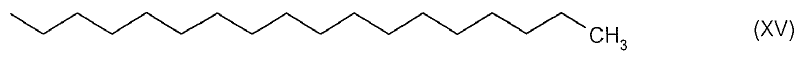

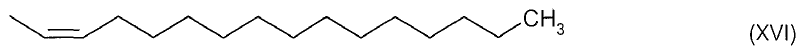

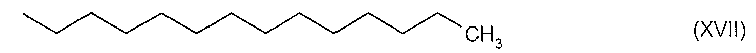

The phospholipid which is a stable vesicle former is selected from 1,2-dioleoyl-sn-glycero-3-phosphocholine (DOPC), 1-palmitoyl-2-docosahexaenoyl-sn-glycero-3-phosphocholine (PDPC), soy phosphatidylcholine, egg phosphatidylcholine, and mixtures thereof. Polar lipids for use in the present subject matter as unstable vesicle forming members are 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-I-serine] (DOPS), a sphingomyelin (cholesterol will form rafts when added to a vesicle formed from a mixture of a sphingomyelin and DOPC), 1,2-dimyristoyl-sn-glycerol, 1-palmitoyl-2-hydroxy-sn-glycero-3-phosphocholine, 1,2-dioleoyl-3-trimethylammonium-propane (DOTAP), and 1,2-dioleoyl-3-dimethylammonium-propane (DODAP).

Other polar lipids useful for the practice of the presently disclosed subject matter as unstable vesicle forming members include phosphatidyl serine (PS), phosphatidyl glycerol (PG), mixed chain phosphatidyl choline (MPC), phosphatidyl ethanol (PE), and phospholipids containing docosahexaenoic acids. Cit-DOPC and cit-DOPC-e are examples of polar lipids useful as unstable vesicle forming members. Phosphatidylcholines, including those having a docosahexaenoic acid in the sn-1 and sn-2 positions (DHPC) may be used. Other diunsaturated lipids, such as diarachidonylphosphatidylcholine (for example 20:4 DOPC : DArPC), dilinolenoylphosphatidylcholine (for example 18:3 DOPC : DLnPC) are also useful. For example, DOPC may be mixed with increasing amounts of DLnPC, DArPC and DHPC during vesicle preparation. Useful ratios include (DOPC:DLnPC, DArPC or DHPC) ranging from 1-1000:1, such as for example 1:1 and 25-500:1, including, 25:1, 50:1, 100:1, and 500:1. Combinations of phospholipids having large mean molecular areas can also be used, such as DOPC:DLnPC:DHPC. Diacylglycerol, a non-lamellar phase lipid, can also be mixed with DOPC. In addition, one can use polyethylene glycol (PEG) with weights of 20 repeats up to 4000 repeats.

In some embodiments, the ratio of the stable vesicle former phospholipid to the polar lipid which is not a stable vesicle former is 1:1 to 500:1, more preferably 10:1 to 100:1 (for example, 50:1). Examples include: DOPC/DOPC-e (1:1); DOPC/POPA (50:1) and DOPC/POPA (1:1). In some embodiments, the lipid vesicle has a ratio of the stable vesicle former to the functionalized lipid of from about 1:1 to about 500:1, in some embodiments a ratio of from about 1:1 to about 200:1 and in some embodiments a ratio of from about 1:1 to about 50:1.

The fusion rate of a lipid vesicle can be altered by changing a variety of factors, such as temperature, ions, lipid concentration, lipid vesicle composition, flow rates, lipid vesicle size, etc. Altering the phospholipid formulation of lipid vesicles can be used to maximize fusion rates as well as minimize toxicity. Four general approaches can be used to alter fusion rates by manipulating lipid composition:
(1) increasing electrostatic interactions;
(2) destabilizing membrane bilayers;
(3) increasing non-bilayer phases; and
(4) creating dissimilar lipid phases.

Electrostatic interactions can be exploited to increase fusion rates. Phospholipids are classified according to their charge (cationic, anionic, and zwitterionic). Many of the cationic phospholipids, such as PE, and anionic phospholipids, such as phosphatidic acid (POPA), do not form closed vesicles at physiologic pH. However, anionic and cationic lipids mixed with zwitterionic phosphatidylcholines can form closed vesicles at physiologic pH.

The plasma membrane of most cells has a net negative charge. Because of this negative charge, there is a layer of counterbalancing ions, typically calcium, magnesium, sodium and potassium, which presents a net positive charge. Taking advantage of the electrostatic interaction between liposomes and plasma membranes, lipid vesicles can be engineered to have a net negative charge, thus maximizing cell-lipid vesicle fusion. However, some cell plasma membranes contain more cationic lipids which are counterbalanced by a anionic ion layer. In these situations, vesicles are engineered to have a net positive charge to maximize cell-lipid fusion.

Plasma membranes contain lipid domains or rafts that are enriched in a particular lipid species. At the boundary of such a membrane raft are regions of dissimilar lipid species. These regions have the potential for instability, affecting how the membrane interacts with other membranes. Several phospholipids are known to increase lipid raft formation, including mixtures of phosphatidylcholines, sphingomyelin, and cholesterol. For example, DOPC, 18:0 sphingomyelin, and cholesterol can be mixed in a 1:1:1 ratio during FUV preparation. Cholesterol preferentially partitions in the sphingomyelin phase, creating regions that are rich in DOPC and poor in cholesterol, and regions that are rich in sphingomyelin and rich in cholesterol.

Changing the physical parameters of fusion, temperature, concentration, ionic strength, can be used to affect fusion rates. By altering temperature, the free energy (G) of the system is altered, leading to different rates of fusion. Increasing lipid vesicle concentration also affects membrane fusion rates, especially at very high concentrations. The fusion period (length of fusion) and the number of fusion periods also affect the amount of encapsulated contents and membrane components delivered by FUVs.

Temperature can also affect fusion rates. Increasing the temperature of the vesicle solution leads to increased kinetic energy of the vesicles and hence increased capability to fuse. Temperature also affects the free diffusion of the vesicles.

While intuitive that increased concentration leads to increased vesicle fusion to some extent, the rate of membrane fusion is not linear. Once FUV lipids occupy the entire available plasma membrane surface, further fusion is limited. The extent of fusion with the plasma membrane affects membrane volume and properties, such as ion permeability and lipid organization. Therefore, when administering FUVs, FUV concentration must be controlled so that the target cells are effectively treated.

The length of time that fusion is allowed to occur helps to control the extent to which encapsulated substances are delivered. To halt fusion, the vesicles are removed (such as by washing with a buffer), or the concentration of the administered vesicles is such that the vesicles are depleted at the end point of the desired time. Fusion can also be optimized such that the total delivery of the vesicles is controlled through one or multiple administrations. For example, if the target fusion period is 120 minutes, two 60-minute periods can be used, or four 30-minute, twelve 10-minute, or twenty four 5- minute fusion periods can be used. Provided that proper equipment is available, 1 minute or less fusion periods can also be accomplished, although these periods are often inconvenient and technically demanding.

In some embodiments, the fusogenic lipid vesicle has a fusion rate of at least about 20 vesicle fusions/second/mm² of cell membrane.

It is desirable in some instances to be able to store lipid vesicles for long periods of time without substantial loss from the vesicles of the selected materials they are carrying and without unintended fusion of the vesicles with one another. More particularly, so as to be useful in certain commercial settings, lipid vesicle preparations should have long enough shelf-lives to allow them to be easily manufactured, shipped, and stored by intermediate and ultimate users under a variety of temperature conditions. Thus, in some embodiments of the presently disclosed subject matter, the lipid vesicles are lyophilized to preserve the vesicles for long-term storage and transportation of the vesicles prior to use.

Techniques for lipid vesicle preservation by lyophilization are generally known in the art. See for example, U.S. Patent Nos. 5,578,320, 5,008,109 and 4,857,319 and U.S. Published Patent Application No. 2006/0045910.

In general, lyophilization (e.g., "freeze-drying") of vesicles comprises the quick freezing of the vesicles followed by thawing the vesicles under a vacuum, which results in removal of water content from the formulations, thereby preserving the vesicles in a stable form. "Lyophilization", as used herein, also refers to dehydration of the vesicles without prior freezing, by simply being placed under reduced pressure.

Storage periods prior to rehydration and activation of the vesicles can be, for example, from about 10 minutes up to about 5 years, including time periods in between.

Since the vesicles can be very rapidly frozen to temperatures of for example -40°C and below and storage times can be extensive, additional components can be added as protective agents to help preserve the vesicles more effectively. For example, the membrane bilayer and other portions of the vesicle, such as the internal vesicular space, can include a saccharide. Thus, the internal and external surfaces of the membrane bilayer can be coated by the saccharide, while the internal space can contain the saccharide. In some embodiments, the saccharide can be incorporated into the vesicle at a ratio (m/m) with the stable vesicle forming member from about 5:1 to 1:5, and in some embodiments at a ratio of about 1:1.

In some embodiments, the saccharide is any water-soluble saccharide, including monosaccharides, disaccharides, and polysaccharides. The saccharide also can include enantiomers, diastereomers, derivatives, and racemic mixtures of one or more saccharides, which are capable of preserving the lipid vesicle, while maintaining the desired fusogenicity. While not wishing to be bound by any particular theory, it is believed that the saccharide prevents vesicle fusion during the dehydration process by binding to the polar head groups of the lipids, displacing water, and creating a glass that surrounds and protects the bilayer membrane from auto-fusion. Upon re-hydration, the saccharide is likely released, thus allowing water stabilization of the bilayer.

Exemplary monosaccharides useful in the preservation procedure include but are not limited to mannose, fructose, or ribose, but preferably not glucose. Exemplary disaccharides include but are not limited to trehalose, lactose, maltose, sucrose, or turanose. Exemplary polysaccharides include but are not limited to hydroxyethylstarch, inulin, or dextran. A preferred saccharide is the disaccharide D-trehalose.

### III. Methods of Decorating Cells with Active agents

The presently disclosed subject matter provides methods of decorating a cell, such as an outer surface of a cell membrane of a cell, with an active agent such as for example therapeutic molecules, including for example polypeptides, aptamers and small molecules. The cell can be a cell of a tissue or an organ (*e.g*., an endothelial cell). The presently disclosed subject matter further includes cells decorated with therapeutic molecules in accordance with the disclosed methods.

The method comprises contacting a cell with a lipid vesicle disclosed herein comprising a functionalized lipid. The functionalized lipid comprises a tether moiety having binding affinity for a ligand portion of an active agent. The cell is contacted with the active agent comprising the ligand portion either simultaneously or sequentially after cell contact with the vesicle. The ligand binds with affinity to the tether moiety, thereby decorating the cell with the active agent.

In some particular embodiments, the method comprises perfusing a tissue or organ with a first composition, wherein the first composition comprises a fusogenic lipid vesicle that includes the functionalized lipid having tether moieties attached thereto and then perfusing the tissue or organ with a second composition, wherein the second composition comprises an active agent, which includes a ligand portion to which the tether moiety has binding specificity. The lipid vesicle fuses with membranes of cells perfused, which results in incorporation of the functionalized lipid and tether moiety into the cell membrane. The tether moiety is then anchored to the outer surface of the cell membrane. Once the tissue is perfused with the second composition comprising the active agent, the active agent then binds with affinity (for example via non-covalent ligand-tether binding) to the cells comprising the embedded tether moieties, resulting in decoration of the cell membranes with active agents.

The functionalized lipid comprises 1,2-dioleoyl-*sn*-glycero-3-{[N(5-amino-1-carboxypentyl)iminodiacetic acid]succinyl}. In some embodiments, the tether moiety is nickel. Further, the ligand portion of the therapeutic molecule is a poly-histidine.

The tether moiety and ligand are chosen based on the binding affinity for one another. In an embodiment, the functionalized lipid comprises 1,2-dioleoyl-*sn*-glycero-3-{[N(5-amino-1-carboxypentyl)iminodiacetic acid]succinyl} chelated with nickel. In these embodiments, the ligand is poly-histidine incorporated into the therapeutic molecule, such as for example, a therapeutic fusion polypeptide.

Further, the present methods provide for the simultaneous or sequential use of multiple different functionalized lipids each comprising different tether moieties. In these embodiments, a plurality of different active agents (each comprising different ligands having binding affinities for the tether moieties) can be targeted to cells of tissues or organs, thereby allowing decoration of cells with a multitude of different active agents, if desired.

The active agent can be a therapeutic molecule selected for protecting the cell, tissue, or organ from IRI induced complement activation or thrombus formation due to endothelial cell damage, and/or immune system rejection of the tissue or organ, if a transplant. The therapeutic molecule can also be selected for treating a cell, tissue, or organ damaged as a result of IRI or immune system rejection. As such, in some embodiments, the therapeutic molecule can be a T cell apoptosis-inducing molecule, a complement inhibitor, a T cell co-stimulatory blockade molecule, a leukocyte infiltration inhibitor, a neointimal hyperplasia inhibitor, an anticoagulant, or a thrombolytic. In some particular embodiments, the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, urokinase, tissue plasminogen activator (tPA), matrix metalloproteinases (MMP), neuropeptide Y (NPY) receptors, and naturally occurring or synthetic glycoproteins or proteoglycans.

In another aspect, the presently disclosed subject matter provides decorated cells, tissues, or organs generated utilizing the disclosed methods and composition. In some embodiments, the decorated cells (*e.g*., endothelial cells), tissues and organs comprise functionalized lipids that include a tether moiety having binding affinity for a ligand. The decorated cells, tissues and organs further comprise an active agent decorating the outside of the cells, wherein the active agent comprises a ligand portion bound to the tether moiety. In some embodiments, the cell, tissue, or organ is an allograft, xenograft, or an autograft. That is, the tissue or organ can be transplanted from one subject to another, or removed from and placed back into the same subject, either at the same or a different location.

### IV. Methods of Treatment

Immunologic tolerance can be defined as the absence of a pathogenic immune response against specific foreign antigens in the absence of ongoing exogenous immunosuppression (*e.g*., with the retention of immune responses against other antigens). The methods described herein safely and effectively down-regulate the immune response of a recipient subject against foreign antigens produced, for example, by a transplanted cell, tissue, or organ. Further, the methods disclosed herein provide inhibition of immune system components responsible for IRI, also a significant concern related to transplant and other procedures resulting in tissue ischemia.

Generally, the methods of decorating cells as described herein can be used as treatment methods to inhibit rejection of a transplanted cell, tissue or organ and/or inhibit IRI to a cell, tissue or organ. In some embodiments, these treatment methods comprise utilizing two compositions (*e.g*., solutions). The first composition comprises a lipid vesicle comprising a functionalized lipid having a tether moiety, as disclosed herein. The cell, tissue, or organ to be treated is perfused with the first composition and the lipid vesicles therein upon contact with cell membranes fuse with the membranes. The first composition containing the fusogenic lipid vesicles can be perfused, for example, intra-arterially (either *in vivo* or *ex vivo*) or immersion and the vesicles allowed to interact with the cell membrane. During this time, at least some of the vesicles fuse with cell membranes, thereby incorporating the functionalized lipids into the lipid bilayer of those cells. Thus, the first solution can decorate the outer layer of the cell membrane with tether moieties. The second composition comprises a therapeutic molecule comprising a ligand portion having binding affinity for the tether moiety. The therapeutic molecule binds with affinity at its ligand to the tether moiety on the outer lipid bilayer of the cells. The second composition also can be perfused, for example, intra-arterially (either *in vivo* or ex *vivo*) and allowed to interact with the decorated cell membranes. The result is the decoration of cells, tissues and organs with numerous copies of the therapeutic molecule.

An advantage of this technology over other systems that attempt incorporation of therapeutic molecules into or onto cells is the longevity of the decoration in, for example, vessels with blood flow. The tether moieties are directly attached to the functionalized phospholipids in the vesicles, which are then incorporated into the lipid bilayer of the cell membrane. Thus, cell-surface therapeutic molecule decoration can remain for as long as the phospholipids remain within the cell membrane.

The fusogenic lipid vesicles that include the functionalized lipids (*e.g*., first composition) and the therapeutic molecules with ligands (*e.g*., second composition) can be administered in any number of ways. For example, a cell, tissue, or organ already harvested from a donor subject can be perfused in the solution. In addition, a cell, tissue, or organ that has already been transplanted into a recipient subject (which can be the same subject as the donor, e.g., CABG) can be decorated by administering the solutions intravenously to the recipient subject. Further, in instances where IRI is a risk (*e.g*., vascular injury or occlusion), the solutions can be administered directly to the site of ischemia, or systemically, such as for example intravenously. Administration can be repeated at regular intervals until (or if) transplant tolerance is induced or the risk of IRI substantially eliminated.

In one embodiment, the active compounds are prepared with carriers that protect the compound against rapid elimination from the body, such as a controlled release formulation, including implants and microencapsulated delivery systems. Biodegradable or biocompatible polymers can be used, such as ethylene vinyl acetate, polyanhydrides, polyglycolic acid, collagen, polyorthoesters, and polylactic acid. Such materials can be obtained commercially from ALZA Corporation (Mountain View, California, U.S.A.) and NOVA Pharmaceuticals, Inc. (Lake Elsinore, California, U.S.A.), or prepared by one of skill in the art.

Dosage is dictated by, and directly depends on, the unique characteristics of the lipid vesicle, which varies with different lipid compositions, the particular desired therapeutic effect, and the route of administration. The specific dose level and frequency for any particular subject or application can be varied. Factors that should be considered, including (1) the temperature at which administration is made and at which fusion is permitted; (2) the ionic environment of the administration site and the ionic strength of the lipid vesicle composition; and (3) the length of time that fusion is permitted. Controlling these factors helps to control the extent to which the functionalized lipids are delivered.

When administering lipid vesicles, vesicle concentration is controlled to effectively treat the target cells while not inhibiting their function by saturating the plasma membranes with vesicle lipids. Preferable concentrations of lipid vesicles, depending on lipid composition, target cell dispersion and volume to be administered may be 0.5 mg/ml-100 mg/ml, such as 0.5 mg/ml, 1 mg/ml, 5 mg/ml, 10 mg/ml, 20 mg/ml, 30 mg/ml, 40 mg/ml, 50 mg/ml, 60 mg/ml, 70 mg/ml, 80 mg/ml, 90 mg/ml and 100 mg/ml.

Vesicle fusion occurring via electrostatic interactions is significantly affected by changes in calcium and/or magnesium concentrations, and to a lesser extent, changes in sodium and/or potassium concentrations. Modulating these ion concentrations either in the compositions used to administer the lipid vesicles or in compositions administered to a target site before or after vesicle administration, affect dosage considerations. Preferably, ion concentrations of 0.01 nM to 1 mM, including 0.1 nM, 1 nM, 10 nM, 100 nM, 1000 nM, 10 micromole/L, and 100 micromoles/L are used. Combinations of these and other ions can also be used.

As noted, regimes of chronic administration or single dosing can be used and are chosen according to the type of treatment, administration route, and type of vesicles. Preferable fusion periods include 1-180 minutes, such as 1, 5, 10, 30, 60, 120 and 180 minutes. To halt fusion, the vesicle is removed (such as by washing with a buffer), or the concentration of vesicles is such that the vesicles are depleted at the end point of the desired time. Fusion can also be optimized such that the total delivery of the vesicles is controlled through one or multiple administrations. For example, if the fusion period is 120 minutes, two 60 minute periods can be used, or four 30 minute periods, twelve 10 minute periods, or 24 five minute fusion periods.

Further with respect to the therapeutic methods of the presently disclosed subject matter, a preferred subject is a vertebrate subject. A preferred vertebrate is warm-blooded; a preferred warm-blooded vertebrate is a mammal. A preferred mammal is most preferably a human. As used herein, the term "subject" includes both human and animal subjects. Thus, veterinary therapeutic uses are provided in accordance with the presently disclosed subject matter.

As such, the presently disclosed subject matter provides for the treatment of mammals such as humans, as well as those mammals of importance due to being endangered, such as Siberian tigers; of economic importance, such as animals raised on farms for consumption by humans; and/or animals of social importance to humans, such as animals kept as pets or in zoos. Examples of such animals include but are not limited to: carnivores such as cats and dogs; swine, including pigs, hogs, and wild boars; ruminants and/or ungulates such as cattle, oxen, sheep, giraffes, deer, goats, bison, and camels; and horses. Also provided is the treatment of birds, including the treatment of those kinds of birds that are endangered and/or kept in zoos, as well as fowl, and more particularly domesticated fowl, *i.e*., poultry, such as turkeys, chickens, ducks, geese, guinea fowl, and the like, as they are also of economic importance to humans. Thus, also provided is the treatment of livestock, including, but not limited to, domesticated swine, ruminants, ungulates, horses (including race horses), poultry, and the like.

### IV.A. Methods of Inhibiting Transplant Rejection

The methods and compositions of the presently disclosed subject matter can be used to prevent transplant rejection in autografts, xenografts and allografts through suppression of immune responses, including for example leukocyte infiltration and activation, and complement activation.

Without harming or pre-treating the recipient subject, the endothelium of an autograft, allograft or xenograft is coated (*i.e*., decorated) with a protective veil consisting of selected therapeutic molecules. In the case of for example T cell-apoptosis inducing polypeptides, such as FasL, donor- and recipient-activated T cells undergo apoptosis when contacting the therapeutic molecules, thereby circumventing rejection. The presently disclosed subject matter allows for the significant reduction, if not elimination, of non-specific immunosuppression therapy after transplantation.

Representative advantages of the presently disclosed subject matter over other immunorejection therapies include: (1) target tissues can be quickly treated to "express" functional exogenous therapeutic molecules in less than an hour; (2) the recipient subject does not require pre-treatment; (3) the materials and procedures are safe for both the treated tissue and the recipient subject; and (4) the therapeutic molecules can remain on the cell surface for at least two weeks, long enough to allow for the development of transplantation tolerance.

In some embodiments, methods of inhibiting rejection of a transplanted tissue or organ in a subject are provided. In some embodiments, the methods comprise contacting the cell, tissue, or organ with a first composition comprising a lipid vesicle, wherein the lipid vesicle comprises a functionalized lipid comprising a tether moiety having binding affinity for a ligand portion of a therapeutic molecule and contacting the cell, tissue, or organ with a second composition comprising the therapeutic molecule comprising the ligand portion, wherein the ligand portion binds the tether moiety. In some embodiments, the cell, tissue, or organ is contacted with the first and second compositions prior to transplant into the subject and in other embodiments, after transplant into the subject.

The functionalized lipid comprises 1,2-dioleoyl-*sn*-glycero-3-{[N(5-amino-1-carboxypentyl)iminodiacetic acid]succinyl}. In some embodiments, the tether moiety is nickel. Further, the ligand portion of the therapeutic molecule is a poly-histidine.

The tether moiety and ligand are chosen based on the binding affinity for one another. In an embodiment, the functionalized lipid comprises 1,2-dioleoyl-*sn*-glycero-3-{[N(5-amino-1-carboxypentyl)iminodiacetic acid]succinyl} chelated with nickel. In these embodiments, the ligand is poly-histidine incorporated into the therapeutic molecule, such as for example, a therapeutic fusion polypeptide.

Further, the present methods provide for the simultaneous or sequential use of multiple different functionalized lipids each comprising different tether moieties. In these embodiments, a plurality of different therapeutic molecules (each comprising different ligands having binding affinities for the tether moieties) can be targeted to cells of tissues or organs, thereby allowing decoration of cells with a multitude of different therapeutic molecules, if desired.

The therapeutic molecule can be selected for protecting the cell, tissue, or organ from immune system rejection of the cell, tissue, or organ at or after transplant. The therapeutic molecule can also be selected for treating a cell, tissue, or organ damaged as a result of immune system rejection. As such, in some embodiments, the therapeutic molecule can be a T cell apoptosis-inducing molecule, a complement inhibitor, a T cell co-stimulatory blockade molecule, a leukocyte infiltration inhibitor, a neointimal hyperplasia inhibitor, an anticoagulant, or a thrombolytic. In some particular embodiments, the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, urokinase, tissue plasminogen activator (tPA), matrix metalloproteinases (MMP), neuropeptide Y (NPY) dummy receptors, and naturally occurring or synthetic glycoproteins or proteoglycans.

### IV.B. Methods of Inhibiting Ischemia-Reperfusion Injury

The presently disclosed subject matter further provides methods of preventing, inhibiting, or treating ischemia-reperfusion injury in a subject. Therapeutic molecules that can inhibit early stages of the immune response observed in IRI, such as complement activation and therapeutic molecules that can inhibit thrombus formation or dissolve thrombi can be decorated onto cells to prevent, inhibit, or treat IRI. In some embodiments, the methods comprise contacting the tissue or organ with a first composition comprising a lipid vesicle, wherein the lipid vesicle comprises a functionalized lipid comprising a tether moiety having binding affinity for a ligand portion of a therapeutic molecule and contacting the tissue or organ with a second composition comprising the therapeutic molecule comprising the ligand portion, wherein the ligand portion binds the tether moiety.

The functionalized lipid comprises 1,2-dioleoyl-*sn*-glycero-3-{[N(5-amino-1-carboxypentyl)iminodiacetic acid]succinyl}. In some embodiments, the tether moiety is nickel. Further, the ligand portion of the therapeutic molecule is a poly-histidine.

The tether moiety and ligand are chosen based on the binding affinity for one another. In one embodiment, the functionalized lipid comprises 1,2-dioleoyl-*sn*-glycero-3-{[N(5-amino-1-carboxypentyl)iminodiacetic acid]succinyl} chelated with nickel. In these embodiments, the ligand is poly-histidine incorporated into the therapeutic molecule, such as for example, a therapeutic fusion polypeptide.

Further, the present methods provide for the simultaneous or sequential use of multiple different functionalized lipids each comprising different tether moieties. In these embodiments, a plurality of different therapeutic molecules (each comprising different ligands having binding affinities for the tether moieties) can be targeted to cells of tissues or organs, thereby allowing decoration of cells with a multitude of different therapeutic molecules, if desired.

As noted, the therapeutic molecule can be selected for protecting, inhibiting, and/or treating the cell, tissue, or organ from IRI. As such, in some embodiments, the therapeutic molecule can be a complement inhibitor, an anticoagulant, or a thrombolytic. In some particular embodiments, the therapeutic molecule is selected from the group consisting of vaccinia virus complement control protein (VCP), complement receptor 1 (CR1), decay accelerating factor (DAF), smallpox inhibitor of complement enzymes (SPICE), compstatin, FUT-175, compound 4077, C1s-INH-248, compound 53, hirudin, small molecule factor Xa inhibitors, small molecule thrombin inhibitors, factor IXa aptamer inhibitor 9.3tC, urokinase, tissue plasminogen activator (tPA) and matrix metalloproteinases (MMP).

### V. Kits

The presently disclosed subject matter further provides kits for decorating endothelial cells. In some embodiments, the kit comprises a lipid vesicle as disclosed herein comprising a functionalized lipid comprising a tether moiety having binding affinity for a ligand portion of an active agent and the active agent comprising the ligand portion. In some embodiments, the lipid vesicle is contained within a first container and the active agent is contained within a second container. The containers can further include other components, including diluents, buffers, and preservatives.

The vesicle and/or the active agent can be preserved, such as for example by lyophilization, to provide for long-term storage of the kit prior to use. In some embodiments, the kit also includes instructions for utilizing the vesicles and active agents for decorating endothelial cells.

The kits can be used to decorate endothelial cells of tissues and organs, which are for example used for transplantation or as active agents administered directly to subjects.

### EXAMPLES

### Materials and Methods for Examples

### Biotin Fusogenic Lipid Vesicle Preparation (comparative example).

1,2-dioleoyl-sn-glycerol-3-phosphocholine (DOPC), 1-palmitoyl-2-0leol-sn-glycerol-3-phosphate (POPA), and/or N-biotinoyl-I,2-dihexadecanoyl-sn-glycero-3-phosphoethanolamine (BDGP) is mixed together in chloroform. The chloroform is removed from the lipid material by exposure to nitrogen gas. The lipid film is vacuum pumped for a period of 12 hours to remove traces of chloroform. The lipid material is hydrated in buffer solution (Ringer's lactate or modified Krebs Henseleit) to a concentration of 25 mg/ml. The buffer and lipid are vortexed for a period of 1 minute to create multilamellar vesicles and placed in a 37°C bath for 5 minutes, and repeated 6 times. The multilamellar vesicles are placed in an ice bath using pulse sonication (40% duty cycle) for a period of 5 minutes. The resultant small, fusogenic unilamellar vesicles (FUVs) are briefly centrifuged to remove traces of titanium from the sonicator probe. FUV size are homogenized by pushing them through an extruder. After FUVs are prepared, a sample is taken to measure the average hydrodynamic radius of FUVs using a Proterion DYNAPRO™ LSD Particle Size Analyzer (Proterion Corp., Piscataway, New Jersey, U.S.A.) to confirm vesicle size.

*Nickel Fusogenic Lipid Vesicle Preparation.* 1,2-dioleoyl-sn-glycerol-3-phosphocholine (DOPC), 1-palmitoyl-2-0leol-sn-glycerol-3-phosphate (POPA), and/or 1,2-dioleoyl-*sn*-glycero-3-{[N(5-amino-1-carboxypentyl)iminodiacetic acid]succinyl} (nickel salt) (DOGS-NTA-Ni) is mixed together in chloroform. The chloroform is removed from the lipid material by exposure to nitrogen gas. The lipid film is vacuum pumped for a period of 12 hours to remove traces of chloroform. The lipid material is hydrated in buffer solution (Ringer's lactate or modified Krebs Henseleit) to a concentration of 25 mg/ml. The buffer and lipid are vortexed for a period of 1 minute to create multilamellar vesicles and placed in a 37°C bath for 5 minutes, and repeated 6 times. The multilamellar vesicles are placed in an ice bath using pulse sonication (40% duty cycle) for a period of 5 minutes. The resultant small, fusogenic unilamellar vesicles (FUVs) are briefly centrifuged to remove traces of titanium from the sonicator probe. FUV size is homogenized by pushing them through an extruder. After FUVs are prepared, a sample is taken to measure the average hydrodynamic radius of FUVs using a Proterion DYNAPRO™ LSD Particle Size Analyzer (Proterion Corp., Piscataway, New Jersey, U.S.A.) to confirm vesicle size.

*Preparation and incubation of HUVEC lines.* HUVECs (Bio Whittaker Corporation) are plated on 12 well dishes and grown to confluence in Clonetics (San Diego, California, U.S.A.) EGM-2 media (modified MCDB 131,5% FBS, 0.04% hydrocortisone, 0.4% hFGF, 0.1 % VEGF, 0.1 % R3-IGF, 0.1 % ascorbic acid, 0.1 % hEGF, 0.1 % GA-I 000, 0.1 % heparin, pH 7.35). Culture media is replaced daily. After 3-5 days, the cells are washed and placed in an experimental media (modified MCDB 131 + 2% heat-inactivated FBS) and incubated for 24 hours. The cells are washed 3x with HBSS (pH 7.35). The effects of endotoxin contamination are reduced by using disposable cellware, media, and buffers low in endotoxin, as well as appropriate control groups.

*Hind Limb Preparation for Ischemia and Reperfusion Injury Studies.* The animals are anesthetized with sodium pentobarbital (60 mg/kg, i.p.) and shaved in the groin area. Access to the flap is by standard groin incision. The femoral artery and vein are dissected from the inguinal ligament to the bifurcation of the inferior epigastric vessels. Murphy's branches are ligated using 8/0 nylon. 0.5 cc of heparin (1,000 UI/ml) are administered through the penile vein of the rat. Following a 10 minute period, the limb, with the exception of the femoral bone and nerves, is separated from the body of the rat. The femoral artery and vein are clamped close to the inguinal ligament and the artery is incised and cannulated with a 24G catheter. The vein is severed, and the limb is perfused with a heparinized Ringer's lactate solution (1 UI/ml) for 10 min. The limb is perfused with solutions containing various FUV formulations with and without functionalized lipids, and after a period of incubation, the FUV solution is flushed. This is followed by a perfusion of a solution containing therapeutic molecules with the appropriate ligand. The solution is allowed to incubate in the limb for a specified period of time and then is flushed with Ringer's lactate. The artery and the vein are repaired and blood flow is reestablished

*Isolation of Endothelial cells from Rat Hind Limb.* The endothelial cells are isolated from the hind limb circulation using collagenase. Briefly, the vessel is perfused briefly with collagenase (2% dissolved in PBS), clamped distally and proximally, and incubated for a period of 20 minutes. The distal clamp is released and the collagenase/cell suspension is isolated. To inhibit the effects of the collagenase on the cells, a 10% FBS solution is added to the isolate. The isolate is centrifuged at 200xg for 10 minutes to pellet the cells. The cells are washed 3x in PBS and then plated for analysis.

*Rat Heart Transplantation: Donor operation.* Rats are anesthetized (sodium pentobarbital 60 *mg*/*kg*, i.p.), prepped and shaved, and 100 VI heparin is injected into the infrahepatic inferior vena cava. The thorax is opened via left and right dorsolateral incisions, and the diaphragm is separated from the anterior chest wall. The right superior vena cava is ligated and cut off on the distal side of the ligation. The first branch of the aorta is ligated with 6-0 silk suture, and the suture is retained to the first branch of the aorta. The left superior vena cava, pulmonary arteries, pulmonary veins, and azygos vein are ligated together with a 4-0 silk suture. Then, the heart is harvested from the donor and stored in cold lactated Ringer's solution. *Recipient operation.* After shaving the neck of the recipient, a right anterolateral neck incision is made. The skin is separated from subcutaneous tissue in order to make a pocket for accommodating the grafted heart loosely. The jugular vein is isolated from its incoming branches and the common carotid artery is ligated away from the subclavian artery as far as possible. Anastomoses between the right superior vena cava of the graft heart and the jugular vein of the recipient as well as between the donor aorta and the common carotid artery of the recipient is performed using 10/0 nylon. After removing all the bulldog clamps and making sure that the beating graft is not congested, the incision is closed with a one layer continuous suture.

*Skin Flap Transplantation: Donor surgery.* Free epigastric groin flaps from ACI rat donors are raised as previously described (Fernandez-Botran et al., 2002, Transplantation, 74:623-629). The flaps measure 5 cm² and include skin, panniculus carnosus muscle, subcutaneous fat, epigastric fat pad, inguinal lymph nodes, and femoral vessels. The animals are anesthetized with sodium pentobarbital (60 mg/kg, i.p.) and shaved in the groin area. Access to the flap is by standard groin incision. The femoral artery and vein are dissected from the inguinal ligament to the bifurcation of the inferior epigastric vessels. The distal ends of the femoral vessels are ligated using 8-0 nylon. The isolated flap is then flushed with heparinized lactated Ringer's solution through the femoral artery for 10 min (venous return is not interrupted). At the end of flushing, the proximal ends of femoral vessels are clamped and the flap raised in its entirety with the epigastric vessels and fat pad. *Recipient surgery.* Nude rat recipients are anesthetized with isoflourane 2-5% and shaved in the ventral aspect of the neck region. The right external carotid artery (ECA) and external jugular vein (EJV) are exposed and the ECA carefully separated from the cervical sympathetic plexus, clamped proximally, and cut. The *EJV* is clamped distally and cut. The flap is then positioned in the neck area with four stay sutures, and vascular anastomoses is performed between the femoral artery and the ECA and the femoral vein and the EJV. The skin is closed using 6-0 nylon.

*Vein Thrombosis Rat Model.* After induction of anesthesia (2-5% isoflourane), a longitudinal incision is made in the right groin of the animal extending 2 cm towards the knee. Through this incision, the right femoral vein is carefully dissected free between the inguinal ligament proximally and the superficial branch epigastric branch distally. the right femoral vein is cannulated for retrograde infusion of treatment solution containing FLVs. A vascular clamp is place at the distal end of the isolated segment to trap the treatment solution. The solution remains in the segment of interest for a predetermined amount of time. The solution then is aspirated with a syringe via the cannula, and the vein is flushed with plain Ringer's lactate solution. Immediately after flushing the vein, a solution containing HN-SA is infused into the vein via the cannula and allowed to remain for 30 min. The solution then is aspirated, the vein is flushed with Ringer's solution, the cannula is removed, the vein is repaired, and the distal vein clamp is released allowing normal blood flow. After 1 hour of reperfusion the animal is placed under a dissecting microscope with a digital video camera that is connected to a computer. Heart rate and blood pressure are monitored. The animal's temperature also is monitored with an intraperitoneal thermometer and maintained at 36-37°C using an adjustable heat pad. A standardized thrombogenic injury is produced in the vein using specially designed forceps that exert a pinch pressure of 1500 g/mm². In other experiments a 2.5% to 10% ferric chloride solution is applied topically to the vein to induce thrombus formation. To observe and measure the pattern of thrombus formation, a transilluminator is placed under the vein while a triocular stereoscopic operating microscope (Carl Zeiss Jena) equipped with an Imaging Source FireWire digital video Camera (DFK 31F03) connected to a video card in a PC is used to visualize and record images. Also a Doppler flow probe is placed on the proximal end of the femoral vein to monitor blood flow and vessel occlusion. The time to complete vessel occlusion is measured.

### Example 1

### Fusogenic Lipid Vesicles

FUVs described herein comprise phospholipids 1,2 dioleoyl-sn-glycerol-3-phosphocholine (DOPC), l-palmitoyl-2-oleoyl-sn-glycerol-3-phosphate (POPA), and dioleoylethylphosphatidylcholine (DOPC-E). The *in vitro* rate of fusion of FUVs to cells (n=6 per FUV type) was determined. Small unilamellar vesicles comprising DOPC alone, DOPC/DOPC-E, or DOPC/POPA were made in a solution containing 1 mM carboxyfluorescein. Human umbilical vein endothelial cells (HUVECs) were incubated with vesicles loaded with carboxyfluorescein, and the rate of delivery over time was quantified. The results demonstrated that the kinetics of carboxyfluorescein delivery to cells was highly dependent upon the composition of the lipid vesicles. DOPC vesicles showed a logarithmic delivery rate in which little or no fusion was observed in the first 30 minutes. This type of delivery rate is more characteristic of endocytosis than of fusion. In contrast, DOPC/DOPC-E gave a much faster initial rate of delivery but a slower final rate. The fastest rate of delivery was found using DOPC/POPA FUVs, which showed an exponential delivery rate in which a significant delivery of carboxyfluorescein was observed within 5 minutes. This type of delivery rate is characteristic of fusion rather than of endocytosis. All three types of vesicles were able to achieve diffuse fluorescent staining of cells within 60 minutes. The DOPC/POPA mixture achieved complete diffuse staining in less than 2 minutes.

### Example 2 (comparative example)

### Lipids from Fusogenic Vesicles Incorporate into Endothelial Cell Membrane When Perfused In Vitro

Fusogenic vesicles composed of DOPC/POPA and biotinylated lipids (N-biotinoyl-1,2-dihexadecanoyl-sn-glycero-3-phosphoethanolamine (BDGP); Molecular Probes, Eugene Oregon, U.S.A.) at a ratio of 1 BDGP/ 5 DOPC phospholipids were prepared. A HUVEC cell culture was then incubated with the fusogenic vesicles for 60 minutes, the culture was washed 3 times, and then the culture was incubated with 1 ml of fluorescent SA (0.5 mg/ml) for 15 minutes. The culture then was washed again 3 times. As a control, the same protocol was followed but biotinylated phospholipids were not incorporated into the fusogenic vesicles from DOPC/POPA. The results of the experiments showed that after washing, the control cells displayed very little fluorescence compared to the brightly lit cells in the group treated with biotinylated FUVs. These results clearly show that the biotin tethers were incorporated into the HUVECs cell membrane and were able to bind the fluorescent labeled SA.

### Example 3

### Lipids from Fusogenic Vesicles Incorporate into Endothelial Cell Membrane When Perfused In Vivo

The ability of FUVs to fuse with endothelial cells of micro vessels was examined using DOPC/POPA and a fluorescent-labeled phospholipid 1,2-dioleoyl-sn-glycero-3-phosphoethanolamine-N-(carboxyfluorescein) (DOPE-CF). Using an isolated (single pedicle) rat cremaster muscle preparation and fluorescent intravital video microscopy, FUV fusion to endothelial cells was observed. After the cremaster was isolated, blood was flushed out with saline, which was followed by an infusion of FUVs made with DOPC/POPA/DOPE-CF. Immediately after infusion, the cremaster's pedicle was clamped for 20 min. The microclamp was then removed, and the cremaster was allowed to reperfuse. As the free fluorescent FUVs were washed away by the blood flow, a visible fluorescent microvascular tree remained in which the edges of vessels showed a halo effect, indicating that fluorescent phospholipids had incorporated into the membranes of endothelial cells. After two hours of reperfusion, the intensity of the fluorescence remained unchanged, suggesting that actual fusion of FUVs to the cell membrane had occurred rather than just aggregating on the cell surface. When the tissues were sectioned and analyzed under the fluorescent microscope, the endothelium clearly stood out from the surrounding tissue with distinct fluorescent staining of the endothelium. Also, while less apparent, the muscle fibers surrounding the vessels were stained with fluorescence, indicating that the fusogenic vesicles had exited the vasculature at some point and had fused with the cells surrounding the vessels.

### Example 4 (comparative example)

### Fusogenic Vesicles Can Be Used to Attach Chimeric Molecules to the Surface of Endothelial Cells In Vivo for a Period of at Least 14 Days

Wistar-Furth (WF) rats were anesthetized and prepared for surgery. The femoral vessels were dissected and the hind limbs separated from the body with the exception of the bones and nerves (femoral and sciatic). The arteries were clamped and cannulated, and the veins were clamped and severed distally from the clamps. The limb was flushed for 10 min with a heparinized Ringer's lactate solution. The limb was perfused with 3 ml of Ringer's lactate solution, and the vessels clamped for 1 hour. After 1 hour, the limb was flushed for 10 minutes with Ringer's lactate solution and then the limb perfused with 1 ml of fluorescent streptavidin (0.1 mg). After 15 minutes of incubation, the limb was again flushed for 10 minutes with Ringer's lactate solution. In Group 1a (negative control), the femoral artery and vein were harvested after fixing with 5% formalin. The vessels were cut in 10 cross-sections 5 microns thick and the intensity of the fluorescence was quantified. In Group 1 b (negative control), the vessels were reanastomosed and the hind limb muscles and skin surgically reattached. The animals were followed for 14 days. The animals were then anesthetized, exanguinated and perfused with a 5% formalin solution. The vessels were dissected, and cut in cross-sections and analyzed as in Group 1 a.

Groups 2a, and 2b were control groups to show that streptavidin does not bind to endothelium that was exposed to non-biotinylated fusogenic vesicles. Rats were prepared in the same way as described above for Groups 1 a, and 1 b, except that the limbs were perfused with 3 ml of fusogenic vesicles that contain only DOPC/POPA (without BDPG). The vessels were clamped. After 1 hour, the limb was flushed as described above and perfused with 1 ml of fluorescent streptavidin (0.1 mg). After 15 minutes of incubation, the limb was again flushed. The amount of fluorescence was measured as described above for Groups 1 a, and 1 b. Very little fluorescence was observed in both groups.

Group 3a, and 3b were the experimental groups to demonstrate that streptavidin does bind to endothelium that was exposed to biotinylated fusogenic vesicles. Rats were prepared as described above for Groups 1 a, and 1 b, except that the limbs were perfused with 3 ml of fusogenic vesicles. The vessels were clamped. After 1 hour, the limb was flushed as described above and perfused with 1ml of fluorescent streptavidin (0.1 mg). After 15 minutes of incubation, the limb was flushed again. The amount of fluorescence was measured as described above for Groups 1 a, and 1 b. A striking difference between experimental animals and control animals was found.

These findings provide evidence that biotinylated phospholipids can be integrated efficiently into the membranes of endothelial cells with the help of fusogenic vesicles. These biotinylated phospholipids can be used as tethers to functionalize the endothelium with streptavidin (SA) and SA-based fusion or chimeric proteins.

### Example 5 (comparative example)

### Optimizing the Biotinylation Ratio

The optimal amount of biotin in fusogenic vesicles was determined. Fusogenic vesicles were prepared with various levels of biotinylation and the vesicles capable of incorporating the largest quantity of biotinylated phospholipids into cultured endothelial cells were determined. HUVEC cells were prepared in Petri dishes and incubated at 37°C in 5% CO₂. FUVs were prepared having the compositions identified in Table 1

**Table 1**

| | BDGP/DOPC | DOPC | POPA | BDGP |
|---|---|---|---|---|
| Formula 1 | 1/5 | 10mg | 0.2518mg | 2.594mg |
| Formula 2 | 1/25 | 10mg | 0.2052mg | 0.259mg |
| Formula 3 | 1/100 | 10mg | 0.2026mg | 0.130mg |
| Formula 4 | 1/500 | 10mg | 0.2005mg | 0.026mg |
| Formula 5 | 1/1000 | 10mg | 0.2002mg | 0.013mg |

| | | | | |
|---|---|---|---|---|
| *Phospholipid Molecular weight:* DOPC(786.1), POPA (696.92), BDGP (1019.45) | | | | |

Three experiments were performed in 96-well plates coated with HUVEC monolayers. In each experiment, 4 wells were assigned to each of the five formulations. The remaining wells were used as controls. The HUVEC monolayer was exposed to 1 ml of fusogenic vesicles prepared according to the formulations described herein and incubated for 20, 40 and 60 min. The cells were washed 3 times with HBSS medium, and incubated with 1 ml of fluorescent streptavidin (Molecular Probes, Eugene, Oregon, U.S.A.)(0.1mg/ml) for 15 min. The cells were washed again 3 times. The amount of fluorescence per well was measured using a microplate reader (Molecular Devices). The process was repeated 3 times to establish which formulation(s) were most efficient at incorporating biotinylated lipids into the membranes of endothelial cells. The results showed that the 1/25 BDGP/DOPC composition gave the greatest biotinylation coverage when incubated for 40 minutes.

### Example 6

### Optimizing the Nickelation Ratio

The optimal amount of nickel in fusogenic vesicles can be determined. Fusogenic vesicles are prepared with various levels of biotinylation and the vesicles capable of incorporating the largest quantity of biotinylated phospholipids into cultured endothelial cells are determined. HUVEC cells can be prepared in Petri dishes and incubated at 37°C in 5% CO₂. FUVs can be prepared having the compositions identified in Table 2

**Table 2**

| | BDGP/DOPC | DOPC | POPA | DOGS-NTA-Ni |
|---|---|---|---|---|
| Formula 1 | 1/5 | 10mg | 0.2518mg | 2.6856mg |
| Formula 2 | 1/25 | 10mg | 0.2052mg | 0.2686mg |
| Formula 3 | 1/100 | 10mg | 0.2026mg | 0.1343mg |
| Formula 4 | 1/500 | 10mg | 0.2005mg | 0.0269mg |
| Formula 5 | 1/1000 | 10mg | 0.2002mg | 0.0134mg |

| | | | | |
|---|---|---|---|---|
| *Phospholipid Molecularweight:* DOPC(786.1), POPA(696.92), DOGS NTA-Ni(1,055.59) | | | | |

Three experiments are performed in 96-well plates coated with HUVEC monolayers. In each experiment, 4 wells are assigned to each of the five formulations. The remaining wells are used as controls. The HUVEC monolayer is exposed to 1ml of fusogenic vesicles prepared according to the formulations described herein and incubated for 1 hour. The cells are washed. 3 times with HBSS medium, and incubated with 1 ml of 6xHis (polyhistidine) Tagged GFP (green fluorescent protein [Qiagen])(0.1mg/ml) for 15 min. The cells are washed again 3 times. The amount of fluorescence per well is measured using a microplate reader (Molecular Devices). The process is repeated 3 times to establish which formulation(s) are most efficient at incorporating biotinylated lipids into the membranes of endothelial cells.

### Example 7 (comparative example)

### Chimeric SA-FasL Polypeptides Retain the Biotin Binding Capacity of Native Streptavidin

To determine whether or not SA-FasL is able to bind to biotinylated phospholipids in endothelial membranes, HUVECs are plated in 96-well plates and allowed to become a monolayer. Experiments are repeated three times. In each experiment, 6 wells are assigned to three biotinylated FUV formulations and the remaining wells are used as controls. HUVEC monolayers are incubated for 1 hour with biotinylated FUVs. Following incubation, the cells are washed 3 times and incubated with 1 ml of SA-FasL (100 ng - M.W. 32 kDa) for 15 minutes. The cells are washed 3 times and are incubated with anti-FasL fluorescent polyclonal antibody (Santa Cruz Biotech) for 20 minutes and washed 3 times. The amount of fluorescence per well is measured using a microplate reader (Molecular Devices). The results are analyzed statistically and compared to control values. For controls, HUVEC monolayers are incubated for 1 hour with biotinylated HBSS, with non-biotinylated fusogenic vesicles and with both.

### Example 8 (comparative example)

### The Efficacy of Bound SA-FasL

Activated T cells that come in contact with Streptavidin-FasL (SA-FasL) tethered to phospholipids on endothelial cells can undergo apoptosis. In three experimental groups, splenocytes from ACI-sensitized WF rats are harvested and exposed to endothelial ACI cells *in vitro.* Next, the WF splenocytes are separated and adoptively transferred to a nude recipient rat (PVG *rnu*/*rnu*). The nude rat is then transplanted with an ACI vascularized skin flap and rejection is monitored.

Group 1 is a negative control group. On day -7, ACI rats are injected with 10⁷ ACI splenocytes. On day-4, nude rats are transplanted with ACT skin flaps in the neck area. Also on day-4, splenocytes are harvested from the autosensitized ACI rats and exposed in a co-culture reaction to ACI endothelial cells. On day 0, the splenocytes are isolated and assessed for apoptotic phenotypes using PI and annexin V-FITC in Flow Cytometry. Ten million (10⁷) splenocytes are injected into the penile vein of the nude rat recipient. The nude rat is followed for 28 days and biopsies are taken of the skin flap on postoperative days 0, 2, 7 and 14, or when signs of rejection or graft-versus-host disease (GVHD) occur. On day 28, the rats are euthanized and samples of the skin flap, as well as the tongue, ear, liver and small bowel are harvested. The latter serves to assess the potential development of GVHD in the nude rat recipients. No rejection of the transplanted ACI skin flaps is expected.

Group 2 is a positive control group. On day-7, WF rats are injected with 10⁷ ACI splenocytes. On day -4, nude rats are transplanted with ACI skin flaps in the neck area. Also on day -4, splenocytes are isolated from the allosensitized WF rats and exposed in a co-culture to ACI endothelial cells. On day 0, the splenocytes are harvested and 10⁷ are injected into the penile vein of the nude rat recipient. The rats are followed and euthanized as described above for Group 1. Strong rejection of the transplanted ACI skin flaps is expected.

Group 3 is the experimental group. On day -7, WF rats are injected with 10⁷ ACI splenocytes. On day -4, nude rats are transplanted with ACI skin flaps in the neck area. Also on day -4, splenocytes are isolated from the allosensitized WF rats and exposed in a co-culture to ACI endothelial cells that are coated with SA-FasL using the procedure described herein. On day 0, the splenocytes are harvested and 10⁷ splenocytes are injected into the penile vein of the nude rat recipient. The rats are followed and euthanized as described above for Group 1.

### Example 9 (comparative example)

### Perfusion with Fusogenic Vesicles and SA-FasL Delays or Prevents Immune Rejection of a Transplanted Rat Heart

A model for heterotopic rat heart transplantation is used to determine whether the endothelium of a heart allograft that is coated with SA-FasL through biotinylated phospholipid tethers is protected against immune rejection *in vivo.* Group I is a control group. WF and ACI rats are prepared and anesthetized on day 0. Hearts are harvested from the ACI donors, prepared as previously described, perfused with cold Ringer's lactate, clamped, incubated for 1 hr, and transplanted subcutaneously to the neck of WF recipients. The vessels used for anastomosis are the carotid artery and jugular vein. The recipients are followed for 12 weeks. Rejection is monitored by palpating the neck and assessing whether the heart is still beating. At 12 weeks or prior to that if the heart is rejected, the recipients are euthanized and blood and hearts are harvested for histological studies. Specifically, the grade of tissue rejection and the presence of SA-FasL on the endothelium are assessed.

Group 2 is an experimental group. Experiments are performed as described for Group 1, with the exception of the solution used to perfuse the ACI hearts. Instead of cold Ringer's lactate as described above, the hearts in Group 2 are perfused with optimized fusogenic vesicles comprising biotinylated lipids in cold Ringer's lactate. Following 1 hr of incubation, 1ml of SA-FasL (100ng) is infused into the coronary arteries and the transplantation of the heart is completed. It is expected that the transplanted hearts in Group 2 survive for a prolonged period of time, whereas the transplanted hearts in Group 1 are completely rejected in approximately 9 days.

### Example 10

### 6xHis-VCP Polypeptides Have Nickel Binding Capacity

This Example pertains to the determination of whether or not the Poly-Histidine moiety in a 6xHis-VCP chimeric polypeptide has Nickel binding capacity. Experiments are performed using cultured HUVECs. A total of twelve groups can be employed. Experiments use 96-well plates in which 8 wells are assigned per group. Experiments are repeated 3 times. A cell-based fluorometric assay is used to quantify the effect of treatment in Groups 2-12. Groups 2-6 are negative controls, and Groups 7-12 are experimental groups in which Nickelated endothelial cells are treated with different concentrations of 6xHis-VCP. The mean percent coverage of 6xHis-VCP per cell is calculated. Group 1 (cell count control). Group 1 is used to determine the number of cells per well. At the end of each experiment, the media from all 96 wells is removed and the plates are frozen. After 24 hours, the plates are thawed and 200µL of CyQUANT GR^{®} dye/cell-lysis buffer (Molecular Probes) is added to the group 1 wells. The plates are then incubated in complete darkness for 5 min at room temperature. The fluorescence of each well is measured using a plate reader in which the excitation and emission filters are set at -480 nm and -520 nm, respectively. The average fluorescence from the 8 wells is determined and used to calculate the cell number per well from a standard curve. Because all 96 wells in each plate are seeded at the same cell density, Group 1 serves to quantify the average number of cells per well.

Groups 2 to 6. Groups 2-6 are negative controls and serve to determine non-specific binding of 6xHis-VCP to cells without nickel tethers, or non-specific binding of anti-VCP antibody to cells that have not been treated with 6xHis-VCP. Cells are incubated with either HBSS, non-nickelated FUVs, or Ni-FUVs.

Group 2 (No treatment control). HUVECs are incubated with HBSS for 30 min, washed three times with HBSS, and incubated with HBSS for another 30 min. At the end of the incubation period, a primary anti-VCP antibody (200µL) is added and allowed to incubate for 30 min. A secondary fluorescent antibody is then added and incubated for 15 min. Fluorescence is quantified. Group 2 serves to measure non-specific binding of anti-VCP antibody to HUVECs receiving no Ni-FUVs or 6xHis-VCP. Cells in Group 2 are expected to exhibit little or no binding of anti-VCP antibody. It is noted that while the cells in this group are incubated with HBSS only, they are washed at 30 minutes in order to expose them to the same experimental procedure as the other groups.

Group 3 (non-specific binding of 6xHis-VCP control). The experimental procedures for Group 3 are the same as those for Group 2 except that HUVECs are incubated with HBSS for 30 min, washed three times with HBSS, and incubated with 200 µL of a 100 ng/mL 6xHis-VCP solution for 30 min. Group 3 serves to measure non-specific binding of 6xHis-VCP to HUVECs. Group 3 are expected to exhibit little or no binding of anti-VCP antibody.

Group 4 (non-specific binding of anti- VCP to cells treated with FUV s control). The experimental procedures for Group 4 are the same as those for Group 2 except that HUVECs are incubated with 200 µL of non-nickelated FUV solution for 30 min, washed three times with HBSS, and incubated with 200 µL of HBSS for another 30 min. Group 3 serves to measure non-specific binding of anti- VCP to HUVECs treated with non-nickelated FUVs. Group 4 are expected to exhibit little or no binding of anti-VCP antibody.

Group 5 (non-specific binding of 6xHis -VCP to cells treated with FUVs control). The experimental procedures for Group 5 are the same as those for Group 2 except that HUVECs are incubated with 200 µL of non-nickelated FUV solution for 30 min, washed three times with HBSS, and incubated with 200 µL of a 100 ng/ml 6xHis -VCP solution for another 30 min. Group 5 serves to measure non-specific binding of 6xHis-VCP to HUVECs treated with non-nickelated FUVs and 6xHis-VCP. Group 5 are expected to exhibit little or no binding of anti-VCP antibody.

Group 6 (non-specific binding of anti VCP to cells treated with Ni-FUV s control). The experimental procedures for Group 6 are the same as those for Group 2 except that HUVECs are incubated with Ni-FUV solution for 30 min, washed three times with HBSS, and incubated with HBSS for another 30 min. Group 6 serves to measure non-specific binding of anti-VCP antibody to HUVECs treated with Ni-FUVs only. Group 6 are expected to exhibit little or no binding of anti-VCP antibody.

Groups 7 to 12 (Experimental groups - specific binding of 6xHis-VCP to Nickel). The experimental procedures for Groups 7-12 are the same as those for Group 2 except that HUVECs are incubated with 200 µL of Ni-FUV solution for 30 min, washed three times with HBSS, and incubated for 30 min with 200 µL of 6xHis-VCP solution at one of the following concentrations: 100 pg/mL (group 7), 1 ng/mL (group 8), 10 ng/mL (group 9), 100 ng/mL (group 10), 1 µg/mL (group 11) or 10 µg/mL (group 12). These groups serve to determine the minimal concentration of 6xHis-VCP required to obtain maximal protein coverage of cell membranes.

### Example 11 (comparative example)

### Construction of Streptavidin-VCP Fusion Protein (SA-VCP)

The constructed polypeptide is a fusion between the vaccinia complement control protein (VCP) and streptavidin (SA) from *Streptomyces avidinii.* The VCP domain mediates the chimeric protein's immunomodulatory effects, and the SA domain is used to target the protein to biotin tethers on the vascular endothelium. The SA-VCP fusion protein was constructed at the genetic level. The fusion gene, containing 865 base pairs of DNA, encodes the fusion protein containing the VCP catalytic domain and the SA binding domain. Initially, the gene sequences encoding the constitutional components of VCP and SA protein were obtained from the NCBI's GenBank database. Upon evaluating the feasibility of fusion binding and the suitable restriction enzymes for expression vector cloning, 32 oligonucleotide primers were ordered from Invitrogen, Inc. The primers comprise forward and reverse fourty-mers with a 13 base pair overlap. The primers were irreversibly phosphorylated with T4-polynucleotide kinase (PNK) in a 95% forward reaction in preparation for covalent attachment. In subsequent ligase chain reactions (LCR) using *Pfu* DNA ligase, the overlapping segments guided the primers to anneal and ligate in the correct order along the regions flanked by specific nucleotide primers. PCR (with *Taq* polymerase) was additionally used to amplify the LCR product for detection by electrophoresis.

This protocol has been used to synthesize and recover the correct base pair length segments for the entire gene construct. PCR techniques are used to ligate the four regions of VCP into the full-length construct. The correct cloning arrangement was verified by DNA sequencing. The gene construct was cloned into *Pischia pastoris* yeast cells using the commercially available pPIC9K vector (Invitrogen). Upon expression, the protein is collected from the culture media and purified using affinity chromatography. Proper expression of the chimeric protein and the purification process was verified by Western Blot analysis.

### Example 12 (comparative example)

### Chimeric SA-VCP Polypeptides Retain the Biotin Binding Capacity of Native Streptavidin

This Example pertains to the determination of whether or not the streptavidin in a SA-VCP chimeric polypeptide retains its biotin-binding capacity. Experiments are performed using cultured HUVECs. A total of twelve groups can be employed. Experiments use 96-well plates in which 8 wells are assigned per group. Experiments are repeated 3 times. A cell-based fluorometric assay is used to quantify the effect of treatment in Groups 2-12. Groups 2-6 are negative controls, and Groups 7-12 are experimental groups in which biotinylated endothelial cells are treated with different concentrations of SA-VCP. The mean percent coverage of SA-VCP per cell is calculated. Group 1 (cell count control). Group 1 is used to determine the number of cells per well. At the end of each experiment, the media from all 96 wells is removed and the plates are frozen. After 24 hours, the plates are thawed and 200µL of CyQUANT GR^{®} dye/cell-lysis buffer (Molecular Probes) is added to the group 1 wells. The plates are then incubated in complete darkness for 5 min at room temperature. The fluorescence of each well is measured using a plate reader in which the excitation and emission filters are set at ∼480 nm and -520 nm, respectively. The average fluorescence from the 8 wells is determined and used to calculate the cell number per well from a standard curve. Because all 96 wells in each plate are seeded at the same cell density, Group 1 serves to quantify the average number of cells per well.

Groups 2 to 6. Groups 2-6 are negative controls and serve to determine nonspecific binding of SA-VCP to cells without biotin tethers, or non-specific binding of anti-VCP antibody to cells that have not been treated with SA-VCP. Cells are incubated with either HBSS, non-biotinylated FLVs, or BioFLVs.

Group 2 (No treatment control). HUVECs are incubated with HBSS for 30 min, washed three times with HBSS, and incubated with HBSS for another 30 min. At the end of the incubation period, a primary anti-VCP antibody (200µL) is added and allowed to incubate for 30 min. A secondary fluorescent antibody is then added and incubated for 15 min. Fluorescence is quantified. Group 2 serves to measure non-specific binding of anti-VCP antibody to HUVECs receiving no BioFLVs or SA-VCP. Cells in Group 2 are expected to exhibit little or no binding of anti-VCP antibody. It is noted that while the cells in this group are incubated with HBSS only, they are washed at 30 minutes in order to expose them to the same experimental procedure as the other groups.

Group 3 (non-specific binding of SA-VCP control). The experimental procedures for Group 3 are the same as those for Group 2 except that HUVECs are incubated with HBSS for 30 min, washed three times with HBSS, and incubated with 200 µL of a 100 ng/mL SA-VCP solution for 30 min. Group 3 serves to measure non-specific binding of SA-VCP to HUVECs. Group 3 are expected to exhibit little or no binding of anti-VCP antibody.

Group 4 (non-specific binding of anti- VCP to cells treated with FUVs control). The experimental procedures for Group 4 are the same as those for Group 2 except that HUVECs are incubated with 200 µL of non-biotinylated FUV solution for 30 min, washed three times with HBSS, and incubated with 200 µL of HBSS for another 30 min. Group 3 serves to measure non-specific binding of anti-VCP to HUVECs treated with non-biotinylated FUVs. Group 4 are expected to exhibit little or no binding of anti-VCP antibody.

Group 5 (non-specific binding of SA-VCP to cells treated with FUVs control). The experimental procedures for Group 5 are the same as those for Group 2 except that HUVECs are incubated with 200 µL of non-biotinylated FUV solution for 30 min, washed three times with HBSS, and incubated with 200 µL of a 100 ng/ml SA-VCP solution for another 30 min. Group 5 serves to measure non-specific binding of SA-VCP to HUVECs treated with non-biotinylated FUVs and SA-VCP. Group 5 are expected to exhibit little or no binding of anti-VCP antibody.

Group 6 (non-specific binding of anti VCP to cells treated with Biotinylated-FUVs control). The experimental procedures for Group 6 are the same as those for Group 2 except that HUVECs are incubated with Biotinylated-FUVs solution for 30 min, washed three times with HBSS, and incubated with HBSS for another 30 min. Group 6 serves to measure non-specific binding of anti-VCP antibody to HUVECs treated with Biotinylated-FUVs only. Group 6 are expected to exhibit little or no binding of anti-VCP antibody.

Groups 7 to 12 (Experimental groups - specific binding of SA-VCP to biotin). The experimental procedures for Groups 7-12 are the same as those for Group 2 except that HUVECs are incubated with 200 µL of Biotinylated-FUV solution for 30 min, washed three times with HBSS, and incubated for 30 min with 200 µL of SA-VCP solution at one of the following concentrations: 100 pg/mL (group 7), 1 ng/mL (group 8), 10 ng/mL (group 9), 100 ng/mL (group 10), 1 µg/mL (group 11) or 10 µg/mL (group 12). These groups serve to determine the minimal concentration of SA-VCP required to obtain maximal protein coverage of cell membranes.

### Example 13 (comparative example)

### Construction of Hirudin-core streptavidin (HN-SA) Protein

This example outlines the construction of an anti-thrombotic chimeric protein designed to work with our Biotinylated-FUVs. The constructed protein is a fusion between natural HN from leech saliva and core streptavidin (SA) from *Streptomyces avidinii.* The HN domain can mediate the chimeric protein's anti-thrombotic effects, and the SA domain can be used to target the protein to biotin tethers on the vascular endothelium. SA tightly binds to biotin, which can be efficiently incorporated on the surface of vascular endothelial cells by the Biotinylated FUVs disclosed herein. The HN-SA fusion protein can be constructed at the genetic level. Two forms of the gene, differing in the order of the SA and HN domains, are created. Each version of the gene, is approximately 700 DNA base pairs in length and contains both HN and SA domains separated by a flexible linker (a standard (Gly₄-Ser)₃ can be used). The sequences for both HN and SA components are disclsoed in the NCBI's Gene bank database. The coding sequences can be codon optimized for optimal expression in *Pischia pastoris.* In addition, restriction sites for swapping out domains and shuttling the entire coding sequence are included. After synthesis, both HN-SA fusion genes are cloned into an expression vector which contains elements for expression, genomic integration, and selection in *Pischia.* After transformation, clones with the highest expression levels are chosen for protein production. Expression is determined by Western blot analysis (using anti-SA antibodies). Upon expression the HN-SA protein is collected from the supernatant and purified by affinity chromatography (also for the SA domain). HN function of each of the two fusion proteins is compared with wild-type HN. After HN-SA is purified various concentrations of the fusion protein or wild type HN is mixed in 1 mL of freshly drawn rat blood and the ecarin clotting time (ECT) is measured using an ECT Test Card (Pharmanetics Inc, Cary,North Carolina, U.S.A.). The curves generated by the two different HN-SA proteins and wild type HN are compared to each other.

### Example 14

### Construction of Hirudin-poly-histidine (HN-6xHis)

This example outlines the construction of an anti-thrombotic chimeric protein designed to work with nickelated-FUVs disclosed herein. The constructed protein is a fusion between natural HN from leech saliva and a six poly-histidine peptide (6xHis). The HN domain can mediate the chimeric protein's anti-thrombotic effects, and the His domain can be used to target the protein to nickel tethers on the vascular endothelium. His tightly binds to nickel, which can be efficiently incorporated on the surface of vascular endothelial cells by the nickelated-FUVs disclosed herein. The HN-6xHis fusion protein can be constructed at the genetic level. Two forms of the gene, differing in the order of the 6xHis and HN domains, can be created. The HN-6xHis gene, is approximately 220 DNA base pairs in length and contains both HN and 6xHis domains, which in some cases is separated by a flexible linker (a standard (Gly₄-Ser)₃.will be used initially). The sequences for HN is disclosed in the NCBl's Gene bank database. The coding sequences can be codon optimized for optimal expression in *Pischia pastoris.* In addition, restriction sites for swapping out domains and shuttling the entire coding sequence are included. After synthesis, both HN-6xHis fusion genes are cloned into an expression vector which contains elements for expression, genomic integration, and selection in *Pischia.* After transformation, clones with the highest expression levels are chosen for protein production. Expression is determined by Western blot analysis (using anti-His antibodies). Upon expression the HN-6xHis protein is collected from the supernatant and purified by affinity chromatography (for the His domain). HN function of each of the two constructed fusion proteins is compared with wild-type HN. After HN-6xHis is purified various concentrations of the fusion protein or wild type HN is mixed in 1 mL of freshly drawn rat blood and the ecarin clotting time (ECT) is measured using an ECT Test Card (Pharmanetics Inc). The curves generated by the two different HN-6xHis proteins and wild type HN are compared to each other.

### Example 15 (comparative example)

### Determination of binding kinetics of HN-SA to biotin tethers

In these experiments the binding kinetics of HN-SA to biotin tethers is quantified. The minimal concentrations of HN-SA required to achieve maximal protein cell coverage is quantified when the maximum number of biotin tethers is displayed on the surface of cultured HUVECs. Six experimental groups can be included. Experiments use 96-well plates in which 8 wells are assigned per group. HUVECS are biotinylated using biotinylated-FUVs made with a combination of DOPC, POPA, and 1,2-dihexadecanoyl-sn-glycero-3-phosphoethanolamine-N-(biotinyl) (BDPG). In each experimental group, HUVECs are treated with different concentrations of HN-SA. A cell-based fluorometric assay is used to quantify the amount of HN-SA bound to the cells. The mean percent coverage of HN-SA per cell is calculated. Several control groups are included to determine the occurrence of any unspecific binding of either HN-SA to non-biotinylated HUVECs or Anti-HN antibody to untreated cells. On each experiment, 8 wells are used to calculate the average number of cells per well using the CyQUANT^{®} Cell proliferation assay kit (Molecular Probes). Experiments are repeated in triplicate. HUVECs are incubated for 30 min with a Biotinylated-FUV solution. The cells then are washed and incubated for another 30 min with HN-SA solution at one of the following concentrations: 100pg/mL (Group 1), 1 ng/mL (Group 2), 10ng/mL (Group 3), 100ng/mL (Group 4), 1*µ*g/mL (Group 5) or 10*µ*g/mL (Group 6). These groups will serve to determine the minimal concentration of HN-SA required to obtain maximal protein coverage of cell membranes. Groups 7, 8 and 9 are negative controls. Group 7 measures non-specific binding of anti-HN antibody to HUVECs receiving no BioFLVs or HN-SA. Cells are incubated with HBSS only and then with FITC-labeled anti-HN monoclonal antibodies (mAb). Group 8 measures non-specific binding of HN-SA to non-biotinylated HUVECs. Cells are incubated with HN-SA solution only and then with FITC-labeled anti-HN mAb. Group 9 ,measures non-specific binding of HN-SA to HUVECs treated with non-biotinylated FLVs. Cells are treated with non-biotinylated FLVs and then with HN-SA solution. Then cells are incubated with FITC-labeled anti-HN mAb.

### Example 16 (comparative example)

### Anti-thrombotic Effectiveness of HN-SA Display

Femoral veins on day 1 are treated with maximal levels of Biotinylated FUVs and HN-SA similarly as described hereinabove. After therapy administration, veins are repaired and the treated segments marked with two sutures. The skin is closed and animals allowed to recover from anesthesia. After 7, 14, 21 or 30 days post-treatment, thrombus formation after "pinch" injury or Ferric chloride treatment is quantified in different animal groups. To determine complete vessel occlusion a Doppler probe is placed on the proximal end of the thrombus to monitor blood flow. All studies are conducted on Sprague-Dawley rats anesthetized with inhaled isoflourane (2-5%). The experimental design for acute efficacy studies of various concentrations HN-SA displayed consists of 2 treatments (Biotinylated-FUVs and non-biotinylated FUVs) x 3 exposure times x 2 vesicle concentrations x 1 HN-SA concentration = 12 experimental groups. The experimental design for the effective longevity studies of HN-SA displayed consists of 2 treatments (Biotinylated FUVs and non-biotinylated FUVs) x 4 time points (7,14,21 and 30 days) x 1 exposure time x 1 vesicle concentration x 1 HN-SA concentration = 8 experimental groups. During thrombus formation studies rat blood pressure, heart rate, respiration and temperature are monitored. At the end of each experiment animals are euthanized and perfused systemically with 5% formalin to pressure-fix all tissues. The treated and contra-lateral femoral veins are harvested for histological and immunohistochemical analysis.

### Example 17

### Construction of Human Tissue Urokinase Plasminogen Activator-Poly Histidine (UK-6xHis)

This example outlines the construction of a thrombolytic chimeric protein designed to work with nickelated-FUVs disclosed herein. The construct involves a nucleic acid vector capable of high expression of a human tissue urokinase plasminogen activator protein (UK) and a six poly-histidine peptide (6xHis). The nucleic acid vector includes synthetic or cDNA-derived DNA fragments encoding a human tissue UK and an amplifiable dominant selectable marker operably linked to one or more regulatory sequences. The regulatory sequences control expression of an mRNA transcript that includes reading frames. The regulatory sequences include promoters, enhancers and other expression control elements. The expression vector is designed for expression of human UK in a mammalian host cell. Commonly used promoters derived from Adenovirus 2 or cytomegalovirus are used. The vector nucleic acid can be introduced into host cells via conventional transformation or transfection techniques used to introduce foreign nucleic acid into a host cell including calcium phosphate or calcium chloride, lipofection or electroporation. The UK domain of the protein will mediate the chimeric protein's thrombolytic effects, and the 6xHis domain will be used to target the protein to nickel tethers on the vascular endothelium. 6xHis tightly binds to nickel, which can be efficiently incorporated on the surface of vascular endothelial cells by the nickelated-FUVs. The UK-6xHis fusion protein is constructed with the 6xHis linked to either the N- and C- terminus via a flexible linker (the standard (Gly₄-Ser)₃.. Two forms of the gene, differing in the order of the 6xHis and UK domains, are created. The UK-6xHis gene, is approximately 1850 DNA base pairs in length and contains both UK and 6xHis domains. The sequence for UK is disclosed in the NCBI's Gene bank database. The coding sequences are codon optimized for optimal expression in Chinese hamster ovarian (CHO) cells (e.g. DG44 or DXB11). In addition, restriction sites for swapping out domains and shuttling the entire coding sequence are included. After transformation, clones with the highest expression levels are chosen for protein production. Expression is determined by Western blot analysis (using anti-His antibodies). Upon expression, the UK-6xHis protein is collected from lysed CHO cells and purified by column chromatography. UK function of each of the two constructed fusion proteins is compared with natural UK. After the two UK-6xHis proteins are purified, fibrinolytic activity is measured using the fibrin-agar plate assay and compared to commercially available UK. The fibrin-agar reagents are prepared in a Dulbecco's phosphate buffer. TPA protease is incubated in the fibrin-agar plates for 48 hours at concentrations ranging from 0.5 - 5.0 µg/mL. Thrombin (36 units/mL in PBS buffer) is mixed with plasminogen (2.5 units/mL in PBS buffer) at 47°C. ME-Agarose 15 mL(5.3 mg/mL in PBS buffer) is added, and is followed by adding 5 mL of fibrinogen (11 mg/mL in PBS buffer) to each 30 mL Falcon plate. Several 3 mm plugs are made on the plates and test solutions containing 10 µL of control and test solutions are added to each plug. The plates are placed in a humidified box and the solutions are allowed to incubate for 48 hours. The diameters of the lytic zones are measured for each sample. A standard curve is constructed using the diameter of a lytic zone produced by the control TPA solutions plotted against the activity units of dilutions. The standard curve is then used to determine the activity and the amount of a UK-6xHis sample.

### Example 18

### Anti-thrombotic Effectiveness of UK-6xHis Display

Femoral veins on day 1 are treated with maximal levels of nickelated FUVs and UK-6xHis. After therapy administration, veins are repaired and the treated segments are marked with two sutures. The skin is closed and animals are allowed to recover from anesthesia. After 7, 14, 21 or 30 days post-treatment, thrombus formation after "pinch" injury or Ferric chloride treatment is quantified in different animal groups. To determine complete vessel occlusion a Doppler probe is placed on the proximal end of the thrombus to monitor blood flow. All studies are conducted on Sprague-Dawley rats anesthetized with inhaled isoflourane (2-5%). The experimental design for acute efficacy studies of various concentrations UK-6xHis displayed consist of 2 treatments (Nickelated FUVs and non- Nickelated FUVs) x 3 exposure times x 2 vesicle concentrations x 1 UK-6xHis concentration = 12 experimental groups. The experimental design for the effective longevity studies of UK-6xHis displayed consist of 2 treatments (Nickelated FUVs and non-Nickelated FUVs) x 4 time points (7,14, 21 and 30 days) x 1 exposure time x 1 vesicle concentration x 1 UK-6xHis concentration = 8 experimental groups. During thrombus formation studies rat blood pressure, heart rate, respiration and temperature are monitored. At the end of each experiment animals are euthanized and perfused systemically with 5% formalin to pressure-fix all tissues. The treated and contra-lateral femoral veins will be harvested for histological and immunohistochemical analysis.

## Claims

1. A lipid vesicle, comprising
(a) a phospholipid which is a stable vesicle former, selected from 1,2-dioleoyl-sn-glycero-3-phosphocholine (DOPC), soy phosphatidylcholine, egg phosphatidylcholine, and a mixture thereof;
(b) at least one unstable vesicle forming member, wherein the unstable vesicle forming member is selected from the group consisting of a phosphatidyl serine (PS), a phosphatidyl glycerol (PG), a phosphatidyl ethanol (PE), a mixed chain phosphatidyl choline (MPC), a diacylglycerol (DAG), a sphingomyelin, 1,2-dioleoyl-3-trimethylammonium-propane (DOTAP), and 1,2-dioleoyl-3-dimethylammonium-propane (DODAP), 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), 1,2-dimyristoyl-sn-glycerol, 1-palmitoyl-2-hydroxy-sn-glycero-3-phosphocholine; and
(c) a functionalized lipid comprising 1,2-dioleoyl-*sn*-glycero-3-{[N(5-amino-1-carboxypentyl)iminodiacetic acid]succinyl}, and a tether moiety attached thereto having binding affinity for a ligand portion of an active agent, wherein the ligand portion is polyhistidine.

2. The lipid vesicle of claim 1, wherein the active agent is a therapeutic molecule selected from the group consisting of a T cell apoptosis-inducing molecule, a complement inhibitor, a T cell co-stimulatory blockade molecule, a leukocyte infiltration inhibitor, a neointimal hyperplasia inhibitor, an anticoagulant, and a thrombolytic.

3. The lipid vesicle of claim 3, wherein the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, factor IXa aptamer inhibitor 9.3tC, urokinase, tissue plasminogen activator (tPA), matrix metalloproteinases (MMP), neuropeptide Y (NPY) dummy receptors, and naturally occurring or synthetic glycoproteins or proteoglycans.

4. The lipid vesicle of claim 1, wherein the lipid vesicle is lyophilized.

5. The lipid vesicle of claim 1, wherein the lipid vesicle has a ratio of the stable vesicle former to the functionalized lipid of from about 1:1 to about 500:1.

6. The lipid vesicle of claim 1, wherein the phospholipid which is a stable vesicle former is 1,2-dioleoyl-sn-glycero-3-phosphocholine (DOPC), wherein the functionalized lipid is 1,2-dioleoyl-*sn*-glycero-3-{[N(5-amino-1-carboxypentyl)iminodiacetic acid]succinyl}, and wherein the unstable vesicle forming member is 1-palmitoyl-2-oleoyl-sn-glycero-3-phosphate (POPA).

7. The lipid vesicle as claimed in any one of claims 1 to 6, wherein the lipid vesicle is provided in a solution.

8. The lipid vesicle as claimed in any one of claims 1 to 7, wherein the ligand portion is polyhistidine.

9. A decorated cell, comprising a functionalized lipid comprising a tether moiety, wherein the tether moiety is bound to a ligand portion of an active agent, wherein the ligand portion is poly-histidine, wherein the functionalized lipid comprises 1,2-dioleoyl-*sn*-glycero-3-{[N(5-amino-1-carboxypentyl)iminodiacetic acid]succinyl}.

10. The decorated cell of claim 9, wherein the decorated cell is an endothelial cell of a tissue or organ.

11. The decorated endothelial cell of claim 9, wherein the tether moiety is non-covalently bound to the ligand portion of the therapeutic molecule.

12. The decorated cell of claim 9, wherein the decorated cell is decorated with a plurality of different therapeutic molecules.

13. The decorated cell of claim 11, wherein the tether moiety is nickel.

14. The decorated cell of claim 9, wherein the active agent is a therapeutic molecule selected from the group consisting of a T cell apoptosis-inducing molecule, a complement inhibitor, a T cell co-stimulatory blockade molecule, a leukocyte infiltration inhibitor, a neointimal hyperplasia inhibitor, an anticoagulant, and a thrombolytic, optionally wherein the therapeutic molecule is selected from the group consisting of FasL, tumor necrosis factor (TNF) receptors-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, factor IXa aptamer inhibitor 9.3tC, urokinase, tissue plasminogen activator (tPA), matrix metalloproteinases (MMP), neuropeptide Y (NPY) dummy receptors, and naturally occurring or synthetic glycoproteins or proteoglycans.

15. A kit comprising:
(a) a lipid vesicle as claimed in any one of claims 1 to 8; and
(b) an active agent comprising a ligand portion provided in a second container.

16. The kit of claim 15, further including instructions describing a method of using the kit for decorating a cell membrane with an active agent, or describing a method of using the kit for inhibiting rejection of a transplanted tissue or organ, or describing a method of using the kit for inhibiting ischemia-reperfusion injury to a tissue or organ.

17. An *ex vivo* method of decorating a cell membrane with an active agent, comprising:
contacting a cell with a lipid vesicle comprising
a phospholipid which is a stable vesicle former, selected from 1,2-dioleoyl-sn-glycero-3-phosphocholine (DOPC), soy phosphatidylcholine, egg phosphatidylcholine, and a mixture thereof;
at least one unstable vesicle forming member, wherein the unstable vesicle forming member is selected from a phosphatidyl serine (PS), a phosphatidyl glycerol (PG), a phosphatidyl ethanol (PE), a mixed chain phosphatidyl choline (MPC), a diacylglycerol (DAG), a sphingomyelin, 1,2-dioleoyl-3-trimethylammonium-propane (DOTAP), and 1,2-dioleoyl-3-dimethylammonium-propane (DODAP), 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), 1,2-dimyristoyl-sn-glycerol, 1-palmitoyl-2-hydroxy-sn-glycero-3-phosphocholine; and
a functionalized lipid comprising 1,2-dioleoyl-*sn*-glycero-3-{[N(5-amino-1-carboxypentyl)iminodiacetic acid]succinyl}, and a tether moiety attached thereto having binding affinity for a ligand portion of an active agent, wherein the ligand portion is poly-histidine; and
contacting the cell with the active agent comprising the ligand portion,
wherein the ligand portion binds the tether moiety.

18. The method of claim 17, wherein the cell is a cell of a tissue or organ, optionally wherein the tissue or organ is perfused with a first composition comprising the lipid vesicle and a second composition comprising the active agent.

19. The method of claim 17, wherein the cell membrane is decorated with a plurality of different active agents.

20. The method of claim 17, wherein the active agent is a therapeutic molecule selected from the group consisting of a T cell apoptosis-inducing molecule, a complement inhibitor, a T cell co-stimulatory blockade molecule, a leukocyte infiltration inhibitor, a neointimal hyperplasia inhibitor, an anticoagulant, and a thrombolytic, optionally wherein the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, factor IXa aptamer inhibitor 9.3tC, urokinase, tissue plasminogen activator (tPA), matrix metalloproteinases (MMP), neuropeptide Y (NPY) dummy receptors, and naturally occurring or synthetic glycoproteins or proteoglycans.

21. The method of claim 17, wherein the lipid vesicle has a ratio of the stable vesicle former to the functionalized lipid of from about 1:1 to about 500:1, or
wherein the phospholipid which is a stable vesicle former is 1,2-dioleoyl-sn-glycero-3-phosphocholine (DOPC), soy phosphatidylcholine, egg phosphatidylcholine, or a mixture thereof, or wherein the unstable vesicle forming member is an unstable vesicle forming polar lipid selected from the group consisting of 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS),a sphingomyelin, 1,2-dimyristoyl-sn-glycerol, 1-palmitoyl-2-hydroxy-sn-glycero-3-phosphocholine, 1,2-dioleoyl-3-trimethylammonium-propane (DOTAP), and 1,2-dioleoyl-3-dimethylammonium-propane (DODAP).

22. A lipid vesicle, comprising
a phospholipid which is a stable vesicle former selected from 1,2-dioleoyl-sn-glycero-3-phosphocholine (DOPC), soy phosphatidylcholine, egg phosphatidylcholine, and a mixture thereof;
at least one unstable vesicle forming member, wherein the unstable vesicle forming member is selected from a phosphatidyl serine (PS), a phosphatidyl glycerol (PG), a phosphatidyl ethanol (PE), a mixed chain phosphatidyl choline (MPC), a diacylglycerol (DAG), a sphingomyelin, 1,2-dioleoyl-3-trimethylammonium-propane (DOTAP), and 1,2-dioleoyl-3-dimethylammonium-propane (DODAP), 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), 1,2-dimyristoyl-sn-glycerol, 1-palmitoyl-2-hydroxy-sn-glycero-3-phosphocholine; and
a functionalized lipid comprising 1,2-dioleoyl-sn-glycero-3-{[N(5-amino-1-carboxypentyl)iminodiacetic acid]succinyl} and a tether moiety attached thereto having binding affinity for a ligand portion of an active agent, wherein the ligand portion is polyhistidine for use in a method of decorating a cell membrane with an active agent, comprising:
(a) contacting a cell with a lipid vesicle comprising a functionalized lipid comprising a tether moiety having binding affinity for a ligand portion of an active agent, wherein the ligand portion is poly-histidine; and
(b) contacting the cell with the active agent comprising the ligand portion,
wherein the ligand portion binds the tether moiety.

23. A lipid vesicle, comprising
a phospholipid which is a stable vesicle former, selected from 1,2-dioleoyl-sn-glycero-3-phosphocholine (DOPC), soy phosphatidylcholine, egg phosphatidylcholine, and a mixture thereof;
at least one unstable vesicle forming member, wherein the unstable vesicle forming member is selected from a phosphatidyl serine (PS), a phosphatidyl glycerol (PG), a phosphatidyl ethanol (PE), a mixed chain phosphatidyl choline (MPC), a diacylglycerol (DAG), a sphingomyelin, 1,2-dioleoyl-3-trimethylammonium-propane (DOTAP), and 1,2-dioleoyl-3-dimethylammonium-propane (DODAP), 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), 1,2-dimyristoyl-sn-glycerol, 1-palmitoyl-2-hydroxy-sn-glycero-3-phosphocholine; and
a functionalized lipid comprising 1,2-dioleoyl-*sn*-glycero-3-{[N(5-amino-1-carboxypentyl)iminodiacetic acid]succinyl} and a tether moiety attached thereto having binding affinity for a ligand portion of an active agent, wherein the ligand portion is polyhistidine for use in a method of inhibiting rejection of a transplanted tissue or organ in a subject, comprising:
(a) contacting the tissue or organ with a first composition comprising a lipid vesicle, wherein the lipid vesicle comprises a functionalized lipid comprising a tether moiety having binding affinity for a ligand portion of a therapeutic molecule, wherein the ligand portion is poly-histidine; and
(b) contacting the tissue or organ with a second composition comprising the therapeutic molecule comprising the ligand portion, wherein the ligand portion binds the tether moiety.

24. The lipid vesicle of claim 23, wherein the tissue or organ is contacted with
the first and second compositions prior to transplant into the subject, or
wherein the tether moiety is non-covalently bound to the ligand portion of the therapeutic molecule.

25. The lipid vesicle of claim 23, wherein the therapeutic molecule is selected from the group consisting of a T cell apoptosis-inducing molecule, a complement inhibitor, a T cell co-stimulatory blockade molecule, a leukocyte infiltration inhibitor, a neointimal hyperplasia inhibitor, an anticoagulant, and a thrombolytic, or
wherein the therapeutic molecule is selected from the group consisting of FasL, tumor necrosis factor (TNF) receptor-1, TNF-related apoptosis inducing ligand (TRAIL) receptor DR4, TRAIL receptor DR5, vaccinia virus complement control protein (VCP), complement receptor 1 (CR1), decay accelerating factor (DAF), compstatin, smallpox inhibitor of complement enzymes (SPICE), cytotoxic T-lymphocyte-associated protein 4 (CTLA-4), anti-CD40L, hirudin, small molecule factor Xa inhibitors, small molecule thrombin inhibitors, factor IXa aptamer inhibitor 9.3tC, urokinase, tissue plasminogen activator (tPA), matrix metalloproteinases (MMP), neuropeptide Y (NPY) dummy receptors, and naturally occurring or synthetic glycoproteins or proteoglycans.

26. The lipid vesicle of claim 23, wherein the lipid vesicle has a ratio of the stable vesicle former to the functionalized lipid of from about 1:1 to about 500:1.

27. The lipid vesicle of claim 23, wherein the unstable vesicle forming member is an unstable vesicle forming polar lipid selected from the group consisting of 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-9 -3-[phospho-l-serine] (DOPS),a sphingomyelin, 1,2-dimyristoyl-sn-glycerol, 1-palmitoyl-2-hydroxy-sn-glycero-3-phosphocholine, 1,2-dioleoyl-3-trimethylammonium-propane (DOTAP), and 1,2-dioleoyl-3-dimethylammonium-propane (DODAP).

28. A lipid vesicle, comprising
a phospholipid which is a stable vesicle former, selected from 1,2-dioleoyl-sn-glycero-3-phosphocholine (DOPC), soy phosphatidylcholine, egg phosphatidylcholine, and a mixture thereof;
at least one unstable vesicle forming member, wherein the unstable vesicle forming member is selected from a phosphatidyl serine (PS), a phosphatidyl glycerol (PG), a phosphatidyl ethanol (PE), a mixed chain phosphatidyl choline (MPC), a diacylglycerol (DAG), a sphingomyelin, 1,2-dioleoyl-3-trimethylammonium-propane (DOTAP), and 1,2-dioleoyl-3-dimethylammonium-propane (DODAP), 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), 1,2-dimyristoyl-sn-glycerol, 1-palmitoyl-2-hydroxy-sn-glycero-3-phosphocholine; and
a functionalized lipid comprising a tether moiety having binding affinity for a ligand portion of an active agent, wherein the ligand portion is poly-histidine, for use in a
method of inhibiting ischemia-reperfusion injury to a tissue or organ, comprising:
(a) contacting the tissue or organ with a first composition comprising a lipid vesicle, wherein the lipid vesicle comprises a functionalized lipid comprising 1,2-dioleoyl-*sn*-glycero-3-{[N(5-amino-1-carboxypentyl)iminodiacetic acid]succinyl} and a tether moiety attached thereto having binding affinity for a ligand portion of a therapeutic molecule, wherein the ligand portion is poly-histidine; and
(b) contacting the tissue or organ with a second composition comprising the therapeutic molecule comprising the ligand portion, wherein the ligand portion binds the tether moiety.

29. The lipid vesicle of claim 28,
wherein the ligand portion of the therapeutic molecule is a poly-histidine, or
wherein the therapeutic molecule is selected from the group consisting of vaccinia virus complement control protein (VCP), complement receptor 1 (CR1), decay accelerating factor (DAF), smallpox inhibitor of complement enzymes (SPICE), compstatin, FUT-175, compound 4077, C1s-INH-248, compound 53, hirudin, small molecule factor Xa inhibitors, small molecule thrombin inhibitors, factor IXa aptamer inhibitor 9.3tC, urokinase, tissue plasminogen activator (tPA) and matrix metalloproteinases (MMP).

30. The lipid vesicle of claim 28, wherein the lipid vesicle has a ratio of the stable vesicle former to the functionalized lipid of from about 1:1 to about 500:1.

31. The lipid vesicle of claim 28, wherein the phospholipid which is a stable vesicle former is 1,2-dioleoyl-sn-glycero-3-phosphocholine (DOPC), soy phosphatidylcholine, egg phosphatidylcholine, or a mixture thereof, or
wherein the unstable vesicle forming member is an unstable vesicle forming polar lipid selected from the group consisting of 1-palmitoyl-2-oleoyl-sn-glycero-3-phosphate (POPA), 1,2-dioleoyl-sn-glycero-3-ethylphosphocholine (DOPC-e), 1,2-dioleoyl-sn-glycero-3-phosphoethanolamine (DOPE), 1,2-dioleoyl-sn-glycero-3-[phospho-1-serine] (DOPS), a sphingomyelin, 1,2-dimyristoyl-sn-glycerol, 1-palmitoyl-2-hydroxy-sn-glycero-3-phosphocholine, 1,2-dioleoyl-3-trimethylammonium-propane (DOTAP), and 1,2-dioleoyl-3-dimethylammonium-propane (DODAP).

32. The lipid vesicle as claimed in any one of claims 22 to 31, wherein the lipid vesicle is provided in a solution.

33. A kit comprising:
(a) a lipid vesicle as claimed in any one of claims 22 to 31; and
(b) an active agent comprising a ligand portion provided in a second container.

34. The kit of claim 33, further including instructions describing a method of using the kit for decorating a cell membrane with an active agent, or describing a method of using the kit for inhibiting rejection of a transplanted tissue or organ, or describing a method of using the kit for inhibiting ischemia-reperfusion injury to a tissue or organ.

## Patentansprüche

1. Lipidvesikel, umfassend
(a) ein Phospholipid, welches ein stabiler Vesikelbildner ist, ausgewählt aus 1,2-Dioleoyl-sn-glycero-3-phosphocholin (DOPC), Soja-Phosphatidylcholin, Ei-Phosphatidylcholin, und einer Mischung davon;
(b) wenigstens einen instabilen Vesikelbildnerteil, wobei der instabile Vesikelbildnerteil ausgewählt wird aus einer Gruppe, bestehend aus einem Phosphatidylserin (PS), einem Phosphatidylglycerol (PG), einem Phosphatidylethanol (PE), einem gemischtkettigen Phosphatidylcholin (MPC), einem Diacylglycerol (DAG), einem Sphingomyelin, 1,2-Dioleoyl-3-trimethylammoniumpropan (DOTAP), und 1,2-Dioleoyl-3-dimethylammoniumpropan (DODAP), 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-1-serin] (DOPS), 1,2-Dimyristoyl-sn-glycerol, 1-Palmitoyl-2-hydroxy-sn-glycero-3-phosphocholin; und
(c) ein funktionalisiertes Lipid, umfassend 1,2-Dioleoyl-sn-glycero-3-{[N(5-amino-1-carboxypentyl)iminodiessigsäure]succinyl} und einen daran angebrachten Halterest mit einer Bindungsaffinität für einen Ligandenteil eines aktiven Stoffes, wobei der Ligandenteil Polyhistidin ist.

2. Lipidvesikel nach Anspruch 1, wobei der aktive Stoff ein therapeutisches Molekül ist, ausgewählt aus der Gruppe, bestehend aus einem T-Zellen-Apoptoseverursachendem Molekül, einem Komplementinhibitor, einem T-Zellen-kostimulierenden Blockademolekül, einem Leukozyteninfiltrationsinhibitor, einem neointimalen Hyperplasieinhibitor, einem Antikoagulant, und einem Thrombolyt.

3. Lipidvesikel nach Anspruch 2, wobei das therapeutische Molekül ausgewählt wird aus einer Gruppe, bestehend aus FasL, Tumornekrosefaktor (TNF) Rezeptor-1, TNF-bezogene Apoptose-verursachender Ligand (TRAIL) Rezeptor DR4, TRAIL Rezeptor DR5, Vacciniaviruskomplement Kontrollprotein (VCP), Komplementrezeptor 1 (CR1), zerfallbeschleunigender Faktor (DAF), Compstatin, Pockeninhibitor eines Komplementenzyms (SPICE), zytotoxisches T-Lymphozyten-assoziiertes Protein 4 (CTLA-4), anti-CD40L, Hirudin, kleine Molekül Faktor-Xa-Inhibitoren, kleine Molekül Thrombininhibitoren, Faktor IXa Aptamerinhibitor 9.3tC, Urokinase, Gewebeplasminogenaktivator (tPA), Matrix-Metallproteinasen (MMP), Neuropeptid Y (NPY), Dummyrezeptoren, und natürlich vorkommende oder synthetische Glycoproteine oder Proteoglykane.

4. Lipidvesikel nach Anspruch 1, wobei das Lipidvesikel lyophilisiert ist.

5. Lipidvesikel nach Anspruch 1, wobei das Lipidvesikel ein Verhältnis von stabilem Vesikelbildner zu funktionalisiertem Lipid von ca. 1:1 bis ca. 500:1 hat.

6. Lipidvesikel nach Anspruch 1, wobei das Phospholipid,welches der stabile Vesikelbildner ist, 1,2-Dioleoyl-sn-glycero-3-phosphocholin (DOPC) ist, wobei das funktionalisierte Lipid 1,2-Dioleoyl-sn-glycero-3-{[N(5-amino-1-carboxypentyl)iminodiessigsäure]succinyl} ist, und wobei der instabile Vesikelbildnerteil 1-Palmitoyl-2-oleoyl-sn-glycero-3-phosphat (POPA) ist.

7. Lipidvesikel nach einem der Ansprüche 1 bis 6, wobei das Lipidvesikel in einer Lösung bereitgestellt wird.

8. Lipidvesikel nach einem der Ansprüche 1 bis 7, wobei der Ligandenteil Polyhistidin ist.

9. Dekorierte Zelle, umfassend ein funktionalisiertes Lipid, umfassend einen Halterest, wobei der Halterest an einen Ligandenteil eines aktiven Stoffes gebunden ist, wobei der Ligandenteil Polyhistidin ist, wobei das funktionalisierte Lipid 1,2-Dioleoyl-sn-glycero-3-{[N(5-amino-1-carboxypentyl)iminodiessigsäure]succinyl} umfasst.

10. Dekorierte Zelle nach Anspruch 9, wobei die dekorierte Zelle eine endotheliale Zelle eines Gewebes oder Organs ist.

11. Dekorierte endotheliale Zelle nach Anspruch 9, wobei der Halterest nicht kovalent an den Ligandenteil des therapeutischen Moleküls gebunden ist.

12. Dekorierte Zelle nach Anspruch 9, wobei die dekorierte Zelle mit einer Vielzahl verschiedener therapeutischer Moleküle dekoriert ist.

13. Dekorierte Zelle nach Anspruch 11, wobei der Halterest Nickel ist.

14. Dekorierte Zelle nach Anspruch 9, wobei der aktive Stoff ein therapeutisches Molekül ist, ausgewählt aus der Gruppe, bestehend aus T-Zellen-Apoptose-verursachendem Molekül, einem Komplementinhibitor, einem T-Zellen-kostimulierenden Blockademolekül, einem Leukozyteninfiltrationsinhibitor, einem neointimalen Hyperplasieinhibitor, einem Antikoagulant, und einem Thrombolyt, optional wobei das therapeutische Molekül ausgewählt wird aus einer Gruppe bestehend aus FasL, Tumornekrosefaktor (TNF) Rezeptor-1, TNF-bezogene Apoptose-verursachender Ligand (TRAIL) Rezeptor DR4, TRAIL Rezeptor DR5, Vacciniaviruskomplement Kontrollprotein (VCP), Komplementrezeptor 1 (CR1), zerfallbeschleunigender Faktor (DAF), Compstatin, Pockeninhibitor eines Komplementenzyms (SPICE), zytotoxisches T-Lymphozyten-assozüertes Protein 4 (CTLA-4), anti-CD40L, Hirudin, kleine Molekül Faktor-Xa-Inhibitoren, kleine Molekül Thrombininhibitoren, Faktor IXa Aptamerinhibitor 9.3tC, Urokinase, Gewebeplasminogenaktivator (tPA), Matrix-Metallproteinasen (MMP), Neuropeptid Y (NPY), Dummyrezeptoren, und natürlich vorkommende oder synthetische Glycoproteine oder Proteoglykane.

15. Kit umfassend:
(a) ein Lipidvesikel wie beansprucht in einem der Ansprüche 1 bis 8; und
(b) einen aktiven Stoff, umfassend einen Ligandenteil, bereitgestellt in einem zweiten Behältnis.

16. Kit nach Anspruch 15, weiter umfassend Anweisungen, welche die Methode zur Verwendung des Kits zur Dekorierung einer Zellmembran mit einem aktiven Stoff beschreiben, oder welche eine Methode zur Verwendung des Kits zur Verhinderung von Transplantatabstoßung eines transplantierten Gewebes oder Organs beschreiben, oder welche eine Methode zur Verwendung des Kits zur Verhinderung von Ischämie-Reperfusionsschaden an einem Gewebe oder Organ beschreiben.

17. *Ex vivo* Verfahren zur Dekorierung einer Zellmembran mit einem aktiven Stoff, umfassend:
in Kontakt bringen einer Zelle mit einem Lipidvesikel, umfassend
ein Phospholipid, welches ein stabiler Vesikelbildner ist, ausgewählt aus 1,2-Dioleoyl-sn-glycero-3-phosphocholin (DOPC), Soja-Phosphatidylcholin, Ei-Phosphatidylcholin, und einer Mischung davon;
wenigstens einen instabilen Vesikelbildnerteil, wobei der instabile Vesikelbildnerteil ausgewählt wird aus einer Gruppe, bestehend aus einem Phosphatidylserin (PS), einem Phosphatidylglycerol (PG), einem Phosphatidylethanol (PE), einem gemischtkettigen Phosphatidylcholin (MPC), einem Diacylglycerol (DAG), einem Sphingomyelin, 1,2-Dioleoyl-3-trimethylammoniumpropan (DOTAP), und 1,2-Dioleoyl-3-dimethylammoniumpropan (DODAP), 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-l-serin] (DOPS), 1,2-Dimyristoyl-sn-glycerol, 1-Palmitoyl-2-hydroxy-sn-glycero-3-phosphocholin; und
ein funktionalisiertes Lipid, umfassend 1,2-Dioleoyl-sn-glycero-3-{[N(5-amino-1-carboxypentyl)iminodiessigsäure]succinyl}, und einen Halterest daran angebracht mit einer Bindungsaffinität für einen Ligandenteil eines aktiven Stoffes, wobei der Ligandenteil Polyhistidin ist; und
in Kontakt bringen einer Zelle mit einem aktiven Stoff, umfassend den Ligandenteil, wobei der Ligandenteil an den Halterest bindet.

18. Verfahren nach Anspruch 17, wobei die Zelle eine Zelle eines Gewebes oder Organs ist, optional wobei das Gewebe oder Organ durchschwemmt ist mit einer ersten Zusammensetzung, umfassend dem Lipidvesikel und einer zweiten Zusammensetzung, umfassend den aktiven Stoff.

19. Verfahren nach Anspruch 17, wobei die Zellmembran mit einer Vielzahl verschiedener aktiver Stoffe dekoriert ist.

20. Verfahren nach Anspruch 17, wobei der aktive Stoff ein therapeutisches Molekül ist, ausgewählt aus der Gruppe, bestehend aus T-Zellen-Apoptose-verursachendem Molekül, einem Komplementinhibitor, einem T-Zellen-kostimulierenden Blockademolekül, einem Leukozyteninfiltrationsinhibitor, einem neointimalen Hyperplasieinhibitor, einem Antikoagulant, und einem Thrombolyt, optional wobei das therapeutische Molekül ausgewählt wird aus einer Gruppe bestehend aus FasL, Tumornekrosefaktor (TNF) Rezeptor-1, TNF-bezogene Apoptose-verursachender Ligand (TRAIL) Rezeptor DR4, TRAIL Rezeptor DR5, Vacciniaviruskomplement Kontrollprotein (VCP), Komplementrezeptor 1 (CR1), zerfallbeschleunigender Faktor (DAF), Compstatin, Pockeninhibitor eines Komplementenzyms (SPICE), zytotoxisches T-Lymphozyten-assoziiertes Protein 4 (CTLA-4), anti-CD40L, Hirudin, kleine Molekül Faktor-Xa-Inhibitoren, kleine Molekül Thrombininhibitoren, Faktor IXa Aptamerinhibitor 9.3tC, Urokinase, Gewebeplasminogenaktivator (tPA), Matrix-Metallproteinasen (MMP), Neuropeptid Y (NPY), Dummyrezeptoren, und natürlich vorkommende oder synthetische Glycoproteine oder Proteoglykane.

21. Verfahren nach Anspruch 17, wobei das Lipidvesikel ein Verhältnis von stabilen Vesikelbildner zu funktionalisiertem Lipid von ca. 1:1 bis ca. 500:1 aufweist, oder
wobei das Phospholipid,welches der stabile Vesikelbildner ist, 1,2-Dioleoyl-sn-glycero-3-phosphocholin (DOPC), Soja-Phosphatidylcholin, Ei-Phosphatidylcholin, oder einer Mischung davon ist, oder wobei der instabile Vesikelbildnerteil ein instabile Vesikel bildendes, polares Lipid ist, ausgewählt aus der Gruppe, bestehend aus 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-l-serin] (DOPS), einem Sphingomyelin, 1,2-Dimyristoyl-sn-glycerol, 1-Palmitoyl-2-hydroxy-sn-glycero-3-phosphocholin, 1,2-Dioleoyl-3-trimethylammoniumpropan (DOTAP), und 1,2-Dioleoyl-3-dimethylammoniumpropan (DODAP).

22. Lipidvesikel, umfassend
ein Phospholipid, welches ein stabiler Vesikelbildner ist, ausgewählt aus 1,2-Dioleoyl-sn-glycero-3-phosphocholin (DOPC), Soja-Phosphatidylcholin, Ei-Phosphatidylcholin, und einer Mischung davon;
wenigstens einen instabilen Vesikelbildnerteil, wobei der instabile Vesikelbildnerteil ausgewählt wird aus einem Phosphatidylserin (PS), einem Phosphatidylglycerol (PG), einem Phosphatidylethanol (PE), einem gemischt kettigen Phosphatidylcholin (MPC), einem Diacylglycerol (DAG), einem Sphingomyelin, 1,2-Dioleoyl-3-trimethylammoniumpropan (DOTAP), und 1,2-Dioleoyl-3-dimethylammoniumpropan (DODAP), 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-l-serin] (DOPS), 1,2-Dimyristoyl-sn-glycerol, 1-Palmitoyl-2-hydroxy-sn-glycero-3-phosphocholin; und
ein funktionalisiertes Lipid, umfassend 1,2-Dioleoyl-sn-glycero-3-{[N(5-amino-1-carboxypentyl)iminodiessigsäure]succinyl}, und einen daran angebrachten Halterest mit einer Bindungsaffinität für einen Ligandenteil eines aktiven Stoffes, wobei der Ligandenteil Polyhistidin ist, zur Verwendung in einem Verfahren zur Dekorierung einer Zellmembran mit einem aktiven Stoff, umfassend
(a) in Kontakt bringen einer Zelle mit einem Lipidvesikel, umfassend ein funktionalisiertes Lipid, umfassend einen Halterest mit Bindungsaffinität für einen Ligandenteil eines aktiven Stoffes, wobei der Ligandenteil Polyhistidin ist; und
(b) in Kontakt bringen einer Zelle mit einem aktiven Stoff, umfassend den Ligandenteil, wobei der Ligandenteil an den Halterest bindet.

23. Lipidvesikel, umfassend
ein Phospholipid, welches ein stabiler Vesikelbildner ist, ausgewählt aus 1,2-Dioleoyl-sn-glycero-3-phosphocholin (DOPC), Soja-Phosphatidylcholin, Ei-Phosphatidylcholin, und einer Mischung davon;
wenigstens einen instabilen Vesikelbildnerteil, wobei der instabile Vesikelbildnerteil ausgewählt wird aus einem Phosphatidylserin (PS), einem Phosphatidylglycerol (PG), einem Phosphatidylethanol (PE), einem gemischt kettigen Phosphatidylcholin (MPC), einem Diacylglycerol (DAG), einem Sphingomyelin, 1,2-Dioleoyl-3-trimethylammoniumpropan (DOTAP), und 1,2-Dioleoyl-3-dimethylammoniumpropan (DODAP), 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-1-serin] (DOPS), 1,2-Dimyristoyl-sn-glycerol, 1-Palmitoyl-2-hydroxy-sn-glycero-3-phosphocholin; und
ein funktionalisiertes Lipid, umfassend 1,2-Dioleoyl-sn-glycero-3-{[N(5-amino-1-carboxypentyl)iminodiessigsäure]succinyl}, und einen daran angebrachten Halterest mit einer Bindungsaffinität für einen Ligandenteil eines aktiven Stoffes, wobei der Ligandenteil Polyhistidin ist, zur Verwendung in einem Verfahren zur Verhinderung von Transplantatabstoßung eines transplantierten Gewebes oder Organs in einem Patienten, umfassend
(a) in Kontakt bringen des Gewebes oder Organs mit einer ersten Zusammensetzung, umfassend ein Lipidvesikel, wobei das Lipidvesikel umfasst ein funktionalisiertes Lipid umfassend einen Halterest mit Bindungsaffinität für einen Ligandenteil eines therapeutischen Moleküls, wobei der Ligandenteil Polyhistidin ist; und
(b) in Kontakt bringen des Gewebes oder Organs mit einer zweiten Zusammensetzung umfassend das therapeutische Molekül umfassend den Ligandenteil, wobei der Ligandenteil an den Halterest bindet.

24. Lipidvesikel nach Anspruch 23, wobei das Gewebe oder Organ vor der Transplantation in den Patienten mit der ersten und zweiten Zusammensetzung in Kontakt gebracht wird, oder
wobei der Halterest nicht kovalent an den Ligandenteil des therapeutischen Moleküls gebunden ist.

25. Lipidvesikel nach Anspruch 23, wobei das therapeutische Molekül ausgewählt wird aus einer Gruppe bestehend aus einem T-Zellen-Apoptose-verursachendem Molekül, einem Komplementinhibitor, einem T-Zellen-kostimulierenden Blockademolekül, einem Leukozyteninfiltrationsinhibitor, einem neointimalen Hyperplasieinhibitor, einem Antikoagulant, und einem Thrombolyt, oder
wobei das therapeutische Molekül ausgewählt wird aus einer Gruppe bestehend aus FasL, Tumornekrosefaktor (TNF) Rezeptor-1, TNF-bezogene Apoptose-verursachender Ligand (TRAIL) Rezeptor DR4, TRAIL Rezeptor DR5, Vacciniaviruskomplement Kontrollprotein (VCP), Komplementrezeptor 1 (CR1), zerfallbeschleunigender Faktor (DAF), Compstatin, Pockeninhibitor eines Komplementenzyms (SPICE), zytotoxisches T-Lymphozyten-assoziiertes Protein 4 (CTLA-4), anti-CD40L, Hirudin, kleine Molekül Faktor-Xa-Inhibitoren, kleine Molekül Thrombininhibitoren, Faktor IXa Aptamerinhibitor 9.3tC, Urokinase, Gewebeplasminogenaktivator (tPA), Matrix-Metallproteinasen (MMP), Neuropeptid Y (NPY), Dummyrezeptoren, und natürlich vorkommende oder synthetische Glycoproteine oder Proteoglykane.

26. Lipidvesikel nach Anspruch 23, wobei das Lipidvesikel ein Verhältnis von stabilen Vesikelbildner zu funktionalisiertem Lipid von ca. 1:1 bis ca. 500:1 hat.

27. Lipidvesikel nach Anspruch 23, wobei der instabile Vesikelbildnerteil ein instabile Vesikel bildendes, polares Lipid ist, ausgewählt aus der Gruppe bestehend aus 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-l-serin] (DOPS), einem Sphingomyelin, 1,2-Dimyristoyl-sn-glycerol, 1-Palmitoyl-2-hydroxy-sn-glycero-3-phosphocholin, 1,2-Dioleoyl-3-trimethylammoniumpropan (DOTAP), und 1,2-Dioleoyl-3-dimethylammoniumpropan (DODAP).

28. Lipidvesikel, umfassend
ein Phospholipid, welches ein stabiler Vesikelbildner ist, ausgewählt aus 1,2-Dioleoyl-sn-glycero-3-phosphocholin (DOPC), Soja-Phosphatidylcholin, Ei-Phosphatidylcholin, und einer Mischung davon;
wenigstens einen instabilen Vesikelbildnerteil, wobei der instabile Vesikelbildnerteil ausgewählt wird aus einem Phosphatidylserin (PS), einem Phosphatidylglycerol (PG), einem Phosphatidylethanol (PE), einem gemischt kettigen Phosphatidylcholin (MPC), einem Diacylglycerol (DAG), einem Sphingomyelin, 1,2-Dioleoyl-3-trimethylammoniumpropan (DOTAP), und 1,2-Dioleoyl-3-dimethylammoniumpropan (DODAP), 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-1-serin] (DOPS), 1,2-Dimyristoyl-sn-glycerol, 1-Palmitoyl-2-hydroxy-sn-glycero-3-phosphocholin; und
ein funktionalisiertes Lipid, umfassend einen Halterest mit einer Bindungsaffinität für einen Ligandenteil eines aktiven Stoffes, wobei der Ligandenteil Polyhistidin ist, zur Verwendung in einem Verfahren zur Verhinderung von Ischämie-Reperfusionsschaden an einem Gewebe oder Organ, umfassend
(a) in Kontakt bringen des Gewebes oder Organs mit einer ersten Zusammensetzung, umfassend ein Lipidvesikel, wobei das Lipidvesikel umfasst ein funktionalisiertes Lipid, umfassend 1,2-Dioleoyl-sn-glycero-3-{[N(5-amino-1-carboxypentyl)iminodiessigsäure]succinyl} und einen daran angebrachten Halterest mit einer Bindungsaffinität für einen Ligandenteil eines therapeutischen Moleküls, wobei der Ligandenteil Polyhistidin ist; und
(b) in Kontakt bringen des Gewebes oder Organs mit einer zweiten Zusammensetzung, umfassend das therapeutische Molekül, umfassend den Ligandenteil, wobei der Ligandenteil an den Halterest bindet.

29. Lipidvesikel nach Anspruch 28,
wobei der Ligandenteil des therapeutischen Moleküls ein Polyhistidin ist, oder
wobei das therapeutische Molekül ausgewählt wird aus einer Gruppe, bestehend aus Vacciniaviruskomplement Kontrollprotein (VCP), Komplementrezeptor 1 (CR1), zerfallbeschleunigender Faktor (DAF), Pockeninhibitor eines Komplementenzyms (SPICE), Compstatin, FUT-175, Verbindung 4077, C1s-INH-248, Verbindung 53, Hirudin, kleine Molekül Faktor-Xa-Inhibitoren, kleine Molekül Thrombininhibitoren, Faktor IXa Aptamerinhibitor 9.3tC, Urokinase, Gewebeplasminogenaktivator (tPA) und Matrix-Metallproteinasen (MMP).

30. Lipidvesikel nach Anspruch 28, wobei das Lipidvesikel ein Verhältnis von stabilem Vesikelbildner zu funktionalisiertem Lipid von ca. 1:1 bis ca. 500:1 aufweist.

31. Lipidvesikel nach Anspruch 28, wobei das Phospholipid, welches ein stabiler Vesikelbildner ist, ausgewählt wird aus 1,2-Dioleoyl-sn-glycero-3-phosphocholin (DOPC), Soja-Phosphatidylcholin, Ei-Phosphatidylcholin, oder einer Mischung davon, oder
wobei der instabile Vesikelbildnerteil ein instabile Vesikel bildendes, polares Lipid ist, ausgewählt aus der Gruppe, bestehend aus 1-Palmitoyl-2-oleoyl-sn-glycero-3-phosphat (POPA), 1,2-Dioleoyl-sn-glycero-3-ethylphosphocholin (DOPC-e), 1,2-Dioleoyl-sn-glycero-3-phosphoethanolamin (DOPE), 1,2-Dioleoyl-sn-glycero-3-[phospho-l-serin] (DOPS), einem Sphingomyelin, 1,2-Dimyristoyl-sn-glycerol, 1-Palmitoyl-2-hydroxy-sn-glycero-3-phosphocholin, 1,2-Dioleoyl-3-trimethylammoniumpropan (DOTAP), und 1,2-Dioleoyl-3-dimethylammoniumpropan (DODAP).

32. Lipidvesikel wie beansprucht in einem der Ansprüche 22 bis 31, wobei das Lipidvesikel in einer Lösung bereitgestellt wird.

33. Kit umfassend:
(a) ein Lipidvesikel, wie beansprucht in einem der Ansprüche 22 bis 31; und
(b) einen aktiven Stoff, umfassend einen Ligandenteil, bereitgestellt in einem zweiten Behältnis.

34. Kit nach Anspruch 33, weiter umfassend Anweisungen, welche die Methode zur Verwendung des Kits zur Dekorierung einer Zellmembran mit einem aktiven Stoff beschreiben, oder welche eine Methode zur Verwendung des Kits zur Verhinderung von Transplantatabstoßung eines transplantierten Gewebes oder Organs beschreiben, oder welche eine Methode zur Verwendung des Kits zur Verhinderung von Ischämie-Reperfusionsschaden an einem Gewebe oder Organ beschreiben.

## Revendications

1. Vésicule lipidique, comprenant :
(a) un phospholipide qui est un formateur de vésicule stable, choisi parmi la 1,2-dioléoyl-sn-glycéro-3-phosphocholine (DOPC), une phosphatidylcholine de soja, une phosphatidylcholine d'oeuf et un de leurs mélanges ;
(b) au moins un élément formateur de vésicule instable, l'élément formateur de vésicule instable étant choisi dans le groupe constitué par une phosphatidyl sérine (PS), un phosphatidyl glycérol (PG), un phosphatidyl éthanol (PE), une phosphatidyl choline à chaîne mixte (MPC), un diacylglycérol (DAG), une sphingomyéline, le 1,2-dioléoyl-3-triméthylammonium-propane (DOTAP), et le 1,2-dioléoyl-3-diméthylammonium-propane (DODAP), le 1-palmitoyl-2-oléoyl-sn-glycéro-3-phosphate (POPA), la 1,2-oléyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), le 1,2-dimyristoyl-sn-glycérol, la 1-palmitoyl-2-hydroxy-sn-glycéro-3-phosphocholine ; et
(c) un lipide fonctionnalisé comprenant le 1,2-dioléoyl-sn-glycéro-3{[acide N(5-amino-1-carboxypentyl)iminodiacétique]succinyle}, et une fraction d'ancrage qui y est attachée ayant une affinité de liaison pour une partie de ligand d'un agent actif, la partie de ligand étant une polyhistidine.

2. Vésicule lipidique selon la revendication 1, dans laquelle l'agent actif est une molécule thérapeutique choisie dans le groupe constitué par une molécule induisant l'apoptose de lymphocyte T, un inhibiteur de complément, une molécule de blocage co-stimulateur de lymphocyte T, un inhibiteur d'infiltration de leucocyte, un inhibiteur d'hyperplasie néointimale, un anticoagulant et un thrombolytique.

3. Vésicule lipidique selon la revendication 3, dans laquelle la molécule thérapeutique est choisie dans le groupe constitué par FasL, le récepteur-1 de facteur de nécrose tumorale (TNF), le récepteur DR4 de ligand induisant l'apoptose apparenté au TNF (TRAIL), le récepteur DR5 de TRAIL, la protéine de contrôle du complément du virus de la vaccine (VCP), le récepteur 1 du complément (CR1), le facteur d'accélération de dégradation (DAF), la compstatine, l'inhibiteur de variole des enzymes du complément (SPICE), la protéine 4 associée aux lymphocytes T cytotoxiques (CTLA-4), l'anti-CD40L, l'hirudine, les inhibiteurs de facteur Xa petites molécules, les inhibiteurs de thrombine petites molécules, l'inhibiteur 9.3tC d'aptamère du facteur IXa, l'urokinase, l'activateur de plasminogène de tissu (tPA), les métalloprotéinases de matrice (MMP), les récepteurs factices de neuropeptide Y (NPY) et les glycoprotéines naturelles ou synthétiques ou protéoglycanes.

4. Vésicule lipidique selon la revendication 1, dans laquelle la vésicule lipidique est lyophilisée.

5. Vésicule lipidique selon la revendication 1, dans la quelle la vésicule lipidique a un rapport du formateur de vésicule stable au lipide fonctionnalisé d'environ 1 : 1 à 500 : 1.

6. Vésicule lipidique selon la revendication 1, dans laquelle le phospholipide qui est un formateur de vésicule stable est la 1,2-dioléoyl-sn-glycéro-3-phosphocholine (DOPC), dans laquelle le lipide fonctionnalisé est le 1,2-dioléoyl-sn-glycéro-3-{[acide N(5-amino-1-carboxypentyl)iminodiacétique]succinyle}, et dans laquelle l'élément formateur de vésicule instable est le 1-palmitoyl-2-oléoyl-sn-glycéro-3-phosphate (POPA).

7. Vésicule lipidique selon l'une quelconque des revendications 1 à 6, dans laquelle la vésicule lipidique est fournie dans une solution.

8. Vésicule lipidique selon l'une quelconque des revendications 1 à 7, dans laquelle la partie de ligand est une polyhistidine.

9. Cellule décorée, comprenant un lipide fonctionnalisé qui comprend une fraction d'ancrage, dans laquelle la fraction d'ancrage est liée à une partie de ligand d'un agent actif, dans laquelle la partie de ligand est une polyhistidine, dans laquelle le lipide fonctionnalisé comprend le 1,2-dioléoyl-sn-glycéro-3-{[acide N(5-amino-1-carboxypentyl)iminodiacétique]succinyle}.

10. Cellule décorée selon la revendication 9, dans laquelle la cellule décorée est une cellule endothéliale d'un tissu ou d'un organe.

11. Cellule décorée endothéliale selon la revendication 9, dans laquelle la fraction d'ancrage est liée de manière non covalente à la partie de ligand de la molécule thérapeutique.

12. Cellule décorée selon la revendication 9, dans laquelle la cellule décorée est décorée avec une pluralité de molécules thérapeutiques différentes.

13. Cellule décorée selon la revendication 11, dans laquelle la fraction d'ancrage est le nickel.

14. Cellule décorée selon la revendication 9, dans laquelle l'agent actif est une molécule thérapeutique choisie dans le groupe constitué par une molécule induisant l'apoptose de lymphocyte T, une molécule de blocage co-stimulateur de lymphocyte T, un inhibiteur d'infiltration de leucocyte, un inhibiteur d'hyperplasie néointimale, un anticoagulant, et un thrombolytique, éventuellement dans laquelle la molécule thérapeutique est choisie dans le groupe constitué par FasL, le récepteur-1 de facteur de nécrose tumorale (TNF), le récepteur DR4 de ligand induisant l'apoptose apparenté au TNF (TRAIL), le récepteur DR5 de TRAIL, la protéine de contrôle du complément de virus de la vaccine (VCP), le récepteur 1 du complément (CR1), le facteur accélérant la dégradation (DAF), la compstatine, l'inhibiteur de la variole des enzymes du complément (SPICE), la protéine 4 associée aux lymphocytes T cytotoxiques (CTLA-4), l'anti-CD40L, l'hirudine, les inhibiteurs de facteur Xa petites molécules, les inhibiteurs de thrombine petites molécules, l'inhibiteur 9.3tC d'aptamère de facteur IXa, l'urokinase, l'activateur du plasminogène de tissu (tPA), les métalloprotéinases de matrice (MMP), les récepteurs factices de neuropeptide Y (NPY) et les glycoprotéines naturelles ou synthétiques ou les protéoglycanes.

15. Kit comprenant :
(a) une vésicule lipidique selon l'une quelconque des revendications 1 à 8 ; et
(b) un agent actif comprenant une partie de ligand fourni dans un second récipient.

16. Kit selon la revendication 15, comprenant en outre des instructions décrivant un procédé d'utilisation du kit pour décorer une membrane cellulaire avec un agent actif, ou décrivant un procédé d'utilisation du kit pour inhiber le rejet d'un tissu ou d'un organe greffé, ou décrivant un procédé d'utilisation du kit pour inhiber la blessure d'ischémie-reperfusion à un tissu ou un organe.

17. Procédé *ex vivo* de décoration d'une membrane cellulaire avec un agent actif, comprenant :
la mise en contact d'une cellule avec une vésicule lipidique comprenant :
un phospholipide qui est un formateur de vésicule stable, choisi parmi la 1,2-dioléoyl-sn-glycéro-3-phosphocholine (DPOC), une phosphatidylcholine de soja, une phosphatidylcholine d'oeuf et un de leurs mélanges ;
au moins un élément formateur de vésicule instable, l'élément formateur de vésicule instable étant choisi dans le groupe constitué par une phosphatidyl sérine (PS), un phosphatidyl glycérol (PG), un phosphatidyl éthanol (PE), une phosphatidyl choline à chaîne mixte (MPC), un diacylglycérol (DAG), une sphingomyéline, le 1,2-dioléoyl-3-triméthylammonium-propane (DOTAP), et le 1,2-dioléoyl-3-diméthylammonium-propane (DODAP), le 1-palmitoyl-2-oléoyl-sn-glycéro-3-phosphate (POPA), la 1,2-oléyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), le 1,2-dimyristoyl-sn-glycérol, la 1-palmitoyl-2-hydroxy-sn-glycéro-3-phosphocholine ; et
un lipide fonctionnalisé comprenant le 1,2-dioléoyl-sn-glycéro-3{[acide N(5-amino-1-carboxypentyl)iminodiacétique]succinyle}, et une fraction d'ancrage qui y est attachée ayant une affinité de liaison pour une partie de ligand d'un agent actif, la partie de ligand étant une polyhistidine ; et
la mise en contact de la cellule avec l'agent actif comprenant la partie de ligand, dans laquelle la partie de ligand se lie à la fraction d'ancrage.

18. Procédé selon la revendication 17, dans lequel la cellule est une cellule d'un tissu ou d'un organe, éventuellement dans lequel le tissu ou l'organe est perfusé avec une première composition comprenant la vésicule lipidique et une seconde composition comprenant l'agent actif.

19. Procédé selon la revendication 17, dans lequel la membrane cellulaire est décorée avec une pluralité d'agents actifs différents.

20. Procédé selon la revendication 17, dans lequel l'agent actif est une molécule thérapeutique choisie dans le groupe constitué par une molécule induisant l'apoptose de lymphocyte T, un inhibiteur du complément, une molécule de blocage co-stimulateur de lymphocyte T, un inhibiteur d'infiltration de leucocyte, un inhibiteur d'hyperplasie néointimale, un anticoagulant, et un thrombolytique, éventuellement dans laquelle la molécule thérapeutique est choisie dans le groupe constitué par FasL, le récepteur-1 de facteur de nécrose tumorale (TNF), le récepteur DR4 de ligand induisant l'apoptose apparenté au TNF (TRAIL), le récepteur DR5 de TRAIL, la protéine de contrôle du complément de virus de la vaccine (VCP), le récepteur 1 du complément (CR1), le facteur accélérant la dégradation (DAF), la compstatine, l'inhibiteur de la variole des enzymes du complément (SPICE), la protéine 4 associée aux lymphocytes T cytotoxiques (CTLA-4), l'anti-CD40L, l'hirudine, les inhibiteurs de facteur Xa petites molécules, les inhibiteurs de thrombine petites molécules, l'inhibiteur 9.3tC d'aptamère de facteur IXa, l'urokinase, l'activateur du plasminogène de tissu (tPA), les métalloprotéinases de matrice (MMP), les récepteurs factices de neuropeptide Y (NPY) et les glycoprotéines naturelles ou synthétiques ou les protéoglycanes.

21. Procédé selon la revendication 17, dans lequel la vésicule lipidique a un rapport du formateur de vésicule stable au lipide fonctionnalisé d'environ 1 : 1 à 500 : 1, ou
dans lequel le lipide qui est un formateur de vésicule stable est la 1,2-dioléoyl-sn-glycéro-3-phosphocholine (DOPC), une phosphatidylcholine de soja, une phosphatidylcholine d'oeuf ou un de leurs mélanges, ou dans lequel l'élément formateur de vésicule instable est un lipide polaire formant une vésicule instable choisi dans le groupe constitué par le 1-palmitoyl-2-oléoyl-sn-glycéro-3-phosphate (POPA), la 1,2-oléyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), une sphingomyéline, le 1,2-dimyristoyl-sn-glycérol, la 1-palmitoyl-2-hydroxy-sn-glycéro-3-phosphocholine, le 1,2-dioléoyl-3-méthylammonium-propane (DOTAP) et le 1,2-dioléoyl-3-diméthylammonium-propane (DODAP).

22. Vésicule lipidique, comprenant :
un phospholipide qui est un formateur de vésicule stable qui est choisi parmi la 1,2-dioléoyl-sn-glycéro-3-phosphocholine (DOPC), une phosphatidylcholine de soja, une phosphatidylcholine d'oeuf et un de leurs mélanges ;
au moins un élément formateur de vésicule instable, dans lequel l'élément formateur de vésicule instable est choisi parmi une phosphatidyl sérine (PS), un phosphatidyl glycérol (PG), un phosphatidyl éthanol (PE), une phosphatidyl choline à chaîne mixte (MPC), un diacylglycérol (DAG), une sphingomyéline, le 1,2-dioléoyl-3-triméthylammonium-propane (DOTAP), et le 1,2-dioléoyl-3-diméthylammonium-propane (DODAP), le 1-palmitoyl-2-oléoyl-sn-glycéro-3-phosphate (POPA), la 1,2-oléyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), le 1,2-dimyristoyl-sn-glycérol, la 1-palmitoyl-2-hydroxy-sn-glycéro-3-phosphocholine ;
et
un lipide fonctionnalisé comprenant le 1,2-dioléoyl-*sn-*glycéro-3{[acide N(5-amino-1-carboxypentyl)iminodiacétique]succinyle}, et une fraction d'ancrage qui y est attachée ayant une affinité de liaison pour une partie de ligand d'un agent actif, la partie de ligand étant une polyhistidine pour une utilisation dans un procédé de décoration d'une membrane cellulaire avec un agent actif, comprenant :
(a) la mise en contact d'un lipide fonctionnalisé comprenant une fraction d'ancrage ayant une affinité de liaison pour une partie de ligand d'un agent actif, la partie de ligand étant une polyhistidine ; et
(b) la mise en contact de la cellule avec l'agent actif comprenant la partie de ligand, dans laquelle la partie de ligand se lie à la fraction d'ancrage.

23. Vésicule lipidique, comprenant un phospholipide qui est un formateur de vésicule stable qui est choisi parmi la 1,2-dioléoyl-sn-glycéro-3-phosphocholine (DOPC), une phosphatidylcholine de soja, une phosphatidylcholine d'oeuf et un de leurs mélanges ;
au moins un élément formateur de vésicule instable, dans lequel l'élément formateur de vésicule instable est choisi parmi une phosphatidyl sérine (PS), un phosphatidyl glycérol (PG), un phosphatidyl éthanol (PE), une phosphatidyl choline à chaîne mixte (MPC), un diacylglycérol (DAG), une sphingomyéline, le 1,2-dioléoyl-3-triméthylammonium-propane (DOTAP), et le 1,2-dioléoyl-3-diméthylammonium-propane (DODAP), le 1-palmitoyl-2-oléoyl-sn-glycéro-3-phosphate (POPA), la 1,2-oléoyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), le 1,2-dimyristoyl-sn-glycérol, la 1-palmitoyl-2-hydroxy-sn-glycéro-3-phosphocholine ;
et
un lipide fonctionnalisé comprenant le 1,2-dioléoyl-*sn-*glycéro-3{[acide N(5-amino-1-carboxypentyl)iminodiacétique]succinyle}, et une fraction d'ancrage qui y est attachée ayant une affinité de liaison pour une partie de ligand d'un agent actif, la partie de ligand étant une polyhistidine pour une utilisation dans un procédé d'inhibition du rejet d'un tissu ou d'un organe greffé chez un sujet, comprenant :
(a) la mise en contact du tissu ou de l'organe avec une première composition comprenant une vésicule lipidique, la vésicule lipidique comprenant un lipide fonctionnalisé qui comprend une fraction d'ancrage ayant une affinité de liaison pour une partie de ligand d'une molécule thérapeutique, la partie de ligand étant une polyhistidine ; et
(b) la mise en contact du tissu ou de l'organe avec une seconde composition comprenant la molécule thérapeutique qui comprend la partie de ligand, la partie de ligand se liant à la fraction d'ancrage.

24. Vésicule lipidique selon la revendication 23, dans laquelle le tissu ou l'organe est mis en contact avec les première et seconde compositions avant la greffe chez le sujet, ou dans laquelle la fraction d'ancrage est liée de manière non covalente à la partie de ligand de la molécule thérapeutique.

25. Vésicule lipidique selon la revendication 23, dans laquelle la molécule thérapeutique est choisie dans le groupe constitué par une molécule induisant l'apoptose de lymphocyte T, un inhibiteur de complément, une molécule de blocage co-stimulateur de lymphocyte T, un inhibiteur d'infiltration de leucocyte, un inhibiteur d'hyperplasie néointimale, un anticoagulant et un thrombolytique, ou
dans laquelle la molécule thérapeutique est choisie dans le groupe constitué par FasL, le récepteur-1 de facteur de nécrose tumorale (TNF), le récepteur DR4 de ligand induisant l'apoptose apparenté au TNF (TRAIL), le récepteur DR5 de TRAIL, la protéine de contrôle du complément du virus de la vaccine (VCP), le récepteur 1 du complément (CR1), le facteur d'accélération de dégradation (DAF), la compstatine, l'inhibiteur de variole des enzymes du complément (SPICE), la protéine 4 associée aux lymphocytes T cytotoxiques (CTLA-4), l'anti-CD40L, l'hirudine, les inhibiteurs de facteur Xa petites molécules, les inhibiteurs de thrombine petites molécules, l'inhibiteur 9.3tC d'aptamère du facteur IXa, l'urokinase, l'activateur de plasminogène de tissu (tPA), les métalloprotéinases de matrice (MMP), les récepteurs factices de neuropeptide Y (NPY) et les glycoprotéines naturelles ou synthétiques ou protéoglycanes.

26. Vésicule lipidique selon la revendication 23, dans laquelle la vésicule lipidique a un rapport du formateur de vésicule stable au lipide fonctionnalisé d'environ 1 : 1 à 500 : 1.

27. Vésicule lipidique selon la revendication 23, dans laquelle l'élément formateur de vésicule instable est un lipide polaire formater de vésicule instable choisi dans le groupe constitué par le 1-palmitoyl-2-oléoyl-sn-glycéro-3-phosphate (POPA), la 1,2-oléoyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), une sphingomyéline, le 1,2-dimyristoyl-sn-glycérol, la 1-palmitoyl-2-hydroxy-sn-glycéro-3-phosphocholine, le 1,2-dioléoyl-3-triméthylammonium-propane (DOTAP), et le 1,2-dioléoyl-3-diméthylammonium-propane (DODAP).

28. Vésicule lipidique, comprenant :
un phospholipide qui est un formateur de vésicule stable, choisi parmi la 1,2-dioléoyl-sn-glycéro-3-phosphocholine (DOPC), une phosphatidylcholine de soja, une phosphatidylcholine d'oeuf et un de leurs mélanges ;
et
au moins un élément formateur de vésicule instable, l'élément formateur de vésicule instable étant choisi dans le groupe constitué par une phosphatidyl sérine (PS), un phosphatidyl glycérol (PG), un phosphatidyl éthanol (PE), une phosphatidyl choline à chaîne mixte (MPC), un diacylglycérol (DAG), une sphingomyéline, le 1,2-dioléoyl-3-triméthylammonium-propane (DOTAP), et le 1,2-dioléoyl-3-diméthylammonium-propane (DODAP), le 1-palmitoyl-2-oléoyl-sn-glycéro-3-phosphate (POPA), la 1,2-oléoyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), le 1,2-dimyristoyl-sn-glycérol, la 1-palmitoyl-2-hydroxy-sn-glycéro-3-phosphocholine ;
et
un lipide fonctionnalisé comprenant une fraction d'ancrage ayant une affinité de liaison pour une partie de ligand d'un agent actif, la partie de ligand étant une polyhistidine, pour une utilisation dans un procédé d'inhibition de blessure d'ischémie-reperfusion à un tissu ou un organe, comprenant :
(a) la mise en contact du tissu ou de l'organe avec une première composition, dans laquelle la vésicule lipidique comprend un lipide fonctionnalisé qui comprend un lipide fonctionnalisé qui comprend le 1,2-dioléoyl-*sn*-glycéro-3-{[acide N(5-amino-1-carboxypentyl)iminodiacétique]succinyle} et une fraction d'ancrage qui y est attachée ayant une affinité de liaison pour une partie de ligand d'une molécule thérapeutique, la partie de ligand étant une polyhistidine ; et
(b) la mise en contact du tissu ou de l'organe avec une seconde composition comprenant la molécule thérapeutique qui comprend la partie de ligand, la partie de ligand se liant à la fraction d'ancrage.

29. Vésicule lipidique selon la revendication 28,
dans la partie de ligand de la molécule thérapeutique est une polyhistidine,
dans laquelle la molécule thérapeutique est choisie dans le groupe constitué par la protéine de contrôle du complément du virus de la vaccine (VCP), le récepteur 1 du complément (CR1), le facteur d'accélération de dégradation (DAF), l'inhibiteur de la variole d'enzymes du complément (SPICE), la compstatine, le FUT-175, le composé 4077, le C1s-INH-248, le composé 53, l'hirudine, les inhibiteurs de facteur Xa petites molécules, les inhibiteurs de thrombine petites molécules, l'inhibiteur 9.3tC d'aptamère de facteur IXa, l'urokinase, l'activateur du plasminogène de tissu (tPA) et les métalloprotéinases de matrice (MMP).

30. Vésicule lipidique selon la revendication 28, dans laquelle la vésicule lipidique a un rapport du formateur de vésicule stable au lipide fonctionnalisé d'environ 1 : 1 à 500 : 1.

31. Vésicule lipidique selon la revendication 28, dans laquelle le phospholipide qui est la 1,2-dioléoyl-sn-glycéro-3-phosphocholine (DOPC), une phosphatidylcholine de soja, une phosphatidylcholine d'oeuf ou un de leurs mélanges, ou
dans lequel l'élément formateur de vésicule instable est un lipide polaire formant une vésicule instable choisi dans le groupe constitué par le 1-palmitoyl-2-oléoyl-sn-glycéro-3-phosphate (POPA), la 1,2-dioléoyl-sn-glycéro-3-éthylphosphocholine (DOPC-e), la 1,2-dioléoyl-sn-glycéro-3-phosphoéthanolamine (DOPE), la 1,2-dioléoyl-sn-glycéro-3-[phospho-1-sérine] (DOPS), une sphingomyéline, le 1,2-dimyristoyl-sn-glycérol, la 1-palmitoyl-2-hydroxy-sn-glycéro-3-phosphocholine, le 1,2-dioléoyl-3-triméthylammonium-propane (DOTAP) et le 1,2-dioléoyl-3-diméthylammonium-propane (DODAP).

32. Vésicule lipidique selon l'une quelconque des revendications 22 à 31, dans laquelle la vésicule lipidique est fournie dans une solution.

33. Kit comprenant :
(a) une vésicule lipidique selon l'une quelconque des revendications 22 à 31 ; et
(b) un agent actif comprenant une partie de ligand fourni dans un second récipient.

34. Kit selon la revendication 33, comprenant en outre des instructions décrivant un procédé d'utilisation du kit pour décorer une membrane cellulaire avec un agent actif, ou décrivant un procédé d'utilisation du kit pour inhiber le rejet d'un tissu ou d'un organe greffé, ou décrivant un procédé d'utilisation du kit pour inhiber une blessure d'ischémie-reperfusion à un tissu ou un organe.
